# EUROPEAN PATENT APPLICATION

(11) **EP 4 501 941 A1**
(43) Date of publication of application: **05.02.2025**
(21) Application number: 23780684.9
(22) Date of filing: 29.03.2023
(51) Int. Cl.: C07K 4/00, A61K 38/03, A61K 38/10, A61K 38/12, A61P 25/00, C07K 7/08, C07K 7/54, C12N 5/071

(54) **PEPTIDE COMPLEX HAVING TRKB BINDING ACTIVITY**

(30) Priority: 30.03.2022 JP 2022057344
(71) Applicant: PeptiDream Inc., Kawasaki-shi, Kanagawa 210-0821 (JP)
(72) Inventor: SUZUKI Yoshinori, Kawasaki-shi Kanagawa 210-0821 (JP); SHIBATA Yoshihiro, Kawasaki-shi Kanagawa 210-0821 (JP)
(74) Representative: van Dam, Vincent
(86) International application number: PCT/JP2023/012777
(87) International publication number: WO 2023/190675

(57) **Abstract**

[Problem] To provide a novel compound having TrkB-binding activity.

[Solution] A peptide complex having TrkB-binding activity comprises a first peptide having an amino acid sequence represented by X1-X2-X3-X4-3Py-W-X5-X6-X7-X8-X9-X10-V-X11-C (SEQ ID NO: 1), or an amino acid sequence in which 1 to 4 amino acids have been substituted, deleted, added or inserted, wherein: X1 and X10 are amino acids having an aromatic ring in the side chain thereof; X2, X9, and X11 are any amino acids; X3 and X5 are secondary amino acids; X4 and X7 are amino acids having an aromatic ring which may be substituted in the side chain thereof; and X6 and X8 are amino acids having a chain alkyl group in the side chain thereof.

## Description

### TECHNICAL FIELD

The present application relates to a novel peptide complex having TrkB binding activity and a pharmaceutical composition comprising the peptide complex. The present application further relates to a novel peptide complex having TrkB agonist activity and a pharmaceutical composition comprising the peptide complex.

### BACKGROUND OF THE INVENTION

TrkB (tropomyosin receptor kinase B) belongs to a family of single-pass transmembrane receptor tyrosine kinases that includes TrkA and TrkC. These receptor kinases mediate the activity of neurotrophins, which is required for neuronal survival and development. Four types of neurotrophins are known: nerve growth factor (NGF), brain-derived neurotrophic factor (BDNF), neurotrophin-3 (NT-3), and neurotrophin-4/5 (NT-4/5), which bind specifically to their high-affinity receptors, TrkA, TrkB, and TrkC. TrkB is a high-affinity receptor of BDNF and is also known to bind to NT-3 and NT-4/5. The binding of BDNF, which is a dimer, to TrkB causes the dimerization of TrkB, which is a receptor, leading to the autophosphorylation of specific tyrosine residues on the receptor and the activation of signal transduction pathways comprising mitogen-activated protein kinase (MAPK), phosphatidylinositol 3-kinase (PI3K), and phospholipase C-γ (PLC-γ). After binding to BDNF, TrkB mediates a plurality of neurotrophin effects including neuronal differentiation and survival. TrkB plays a major role in the survival, differentiation, and function of neurons. Thus, TrkB agonists are expected to be used in the treatment of many neurodegenerative disorders and neuropsychiatric disorders.

BDNF, which is a naturally occurring TrkB agonist, has been used in clinical trials as a therapeutic drug for the above disorders, but due to pharmacokinetic problems and other problems, there has been no progress in research and development since then. In recent years, TrkB agonist antibodies (Patent Literatures 2 and 5; Non-Patent Literatures 3 and 4), low-molecular-weight compounds (Patent Literature 8; Non-Patent Literatures 1 and 2), and peptides (Patent Literature 9) have been reported as alternatives to BDNF.

### PRIOR LITERATURES

### PATENT LITERATURES

Patent Literature 1: Japanese Unexamined Patent Application Publication (Translation of PCT Application) No. 2010-513461
Patent Literature 2: Japanese Unexamined Patent Application Publication (Translation of PCT Application) No. 2011-510021
Patent Literature 3: Japanese Unexamined Patent Application Publication (Translation of PCT Application) No. 2018-522016
Patent Literature 4: Japanese Unexamined Patent Application Publication (Translation of PCT Application) No. 2019-517787
Patent Literature 5: Japanese Unexamined Patent Application Publication (Translation of PCT Application) No. 2020-513806
Patent Literature 6: Japanese Unexamined Patent Application Publication (Translation of PCT Application) No. 2021-507677
Patent Literature 7: Japanese Unexamined Patent Application Publication (Translation of PCT Application) No. 2021-527070
Patent Literature 8: Japanese Patent No. 5946454
Patent Literature 9: WO2022/009992

### NON-PATENT LITERATURES

Non-Patent Literature 1: PLOS ONE 2014, 9, e87923.
Non-Patent Literature 2: Sci. Signal. 2017, 10, eaal1670.
Non-Patent Literature 3: PNAS, 115, E7023.
Non-Patent Literature 4: Neurobiology of Disease 2019, 132, 104590.

### DISCLOSURE OF THE INVENTION

### PROBLEMS TO BE SOLVED BY THE INVENTION

Therefore, the present invention aims to provide a novel compound having TrkB binding activity. Additionally, the present invention aims to provide a novel compound having TrkB agonist activity.

### SOLUTIONS TO THE PROBLEMS

The first invention in this specification relates to a peptide complex.

The peptide complex comprises a first peptide and has TrkB binding activity.

The first peptide has an amino acid sequence represented by X¹-X²-X³-X⁴-3Py-W-X⁵-X⁶-X⁷-X⁸-X⁹-X¹⁰-V-X¹¹-C (SEQ ID NO: 1) or consists of an amino acid sequence with substitution, deletion, addition, or insertion of 1 to 4 amino acids in the amino acid sequence represented by SEQ ID NO: 1.

X¹ is an amino acid having an aromatic ring in a side chain.

X² is any amino acid.

X³ is a secondary amino acid.

X⁴ is an amino acid having an aromatic ring in a side chain, wherein the aromatic ring may be substituted.

X⁵ is a secondary amino acid.

X⁶ is an amino acid having a chain alkyl group in a side chain.

X⁷ is an amino acid having an aromatic ring in a side chain, wherein the aromatic ring may be substituted.

X⁸ is an amino acid having a chain alkyl group in a side chain.

X⁹ is any amino acid.

X¹⁰ is an amino acid having an aromatic ring in a side chain.

X¹¹ is any amino acid.

### EFFECTS OF THE INVENTION

This invention can provide a novel compound having TrkB binding activity. This invention can also further provide a novel compound having TrkB agonist activity.

### BRIEF DESCRIPTION OF THE DRAWINGS

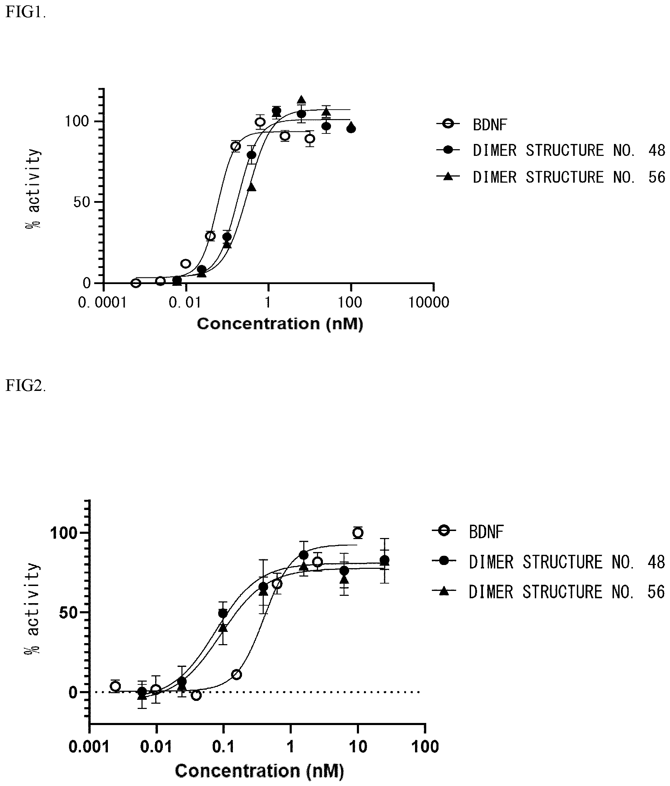

FIG. 1 shows measurement results of TrkB agonist activity by a pERK1/2 AlphaLISA assay using the peptide complex of the present invention and a brain-derived neurotrophic factor (BDNF). In FIG. 1, the white circle represents the brain-derived neurotrophic factor, the black circle represents a peptide complex having TrkB agonist activity (a complex of a peptide (peptide sequence No. 15) and a linker (linker structure No. 7) (dimer structure No. 48 in Table 3)), and the black triangle represents a peptide complex having TrkB agonist activity (a complex of a peptide (peptide sequence No. 17) and a linker (linker structure No. 7) (dimer structure No. 56 in Table 3)). The horizontal axis shows the concentration (nM) of the peptide complex or the brain-derived neurotrophic factor, and the vertical axis shows the relative value of the activation signal when the maximum value of the activation signal induced by the brain-derived neurotrophic factor is 100.
FIG. 2 shows measurement results of TrkB agonist activity by an NFAT assay using the peptide complex of the present invention and a brain-derived neurotrophic factor (BDNF). In FIG. 2, the white circle represents the brain-derived neurotrophic factor, the black circle represents a peptide complex having TrkB agonist activity (a complex of a peptide (peptide sequence No. 15) and a linker (linker structure No. 7) (dimer structure No. 48 in Table 3)), and the black triangle represents a peptide complex having TrkB agonist activity (a complex of a peptide (peptide sequence No. 17) and a linker (linker structure No. 7) (dimer structure No. 56 in Table 3)). The horizontal axis shows the concentration (nM) of the peptide complex or the brain-derived neurotrophic factor, and the vertical axis shows the relative value of the activation signal when the maximum value of the activation signal induced by the brain-derived neurotrophic factor is 100.

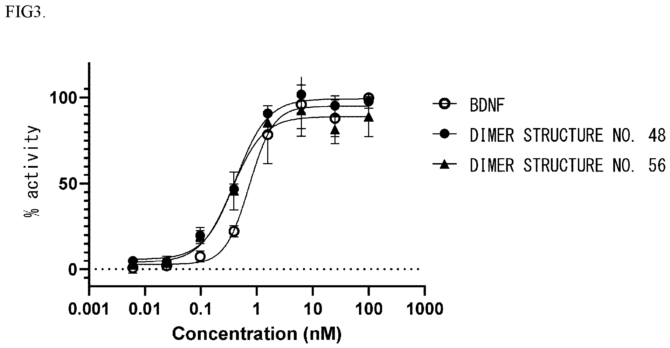

FIG. 3 shows measurement results of TrkB agonist activity by a PathHunter assay using the peptide complex of the present invention and a brain-derived neurotrophic factor (BDNF). In FIG. 3, the white circle represents the brain-derived neurotrophic factor, the black circle represents a peptide complex having TrkB agonist activity (a complex of a peptide (peptide sequence No. 15) and a linker (linker structure No. 7) (dimer structure No. 48 in Table 3)), and the black triangle represents a peptide complex having TrkB agonist activity (a complex of a peptide (peptide sequence No. 17) and a linker (linker structure No. 7) (dimer structure No. 56 in Table 3)). The horizontal axis shows the concentration (nM) of the peptide complex or the brain-derived neurotrophic factor, and the vertical axis shows the relative value of the activation signal when the maximum value of the activation signal induced by the brain-derived neurotrophic factor is 100.

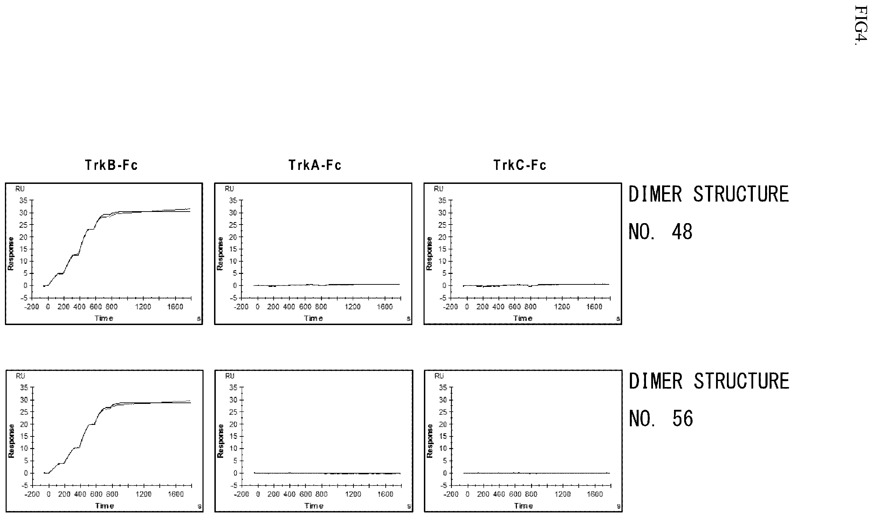

FIG. 4 shows measurement results of molecular interactions with TrkA, TrkB, and TrkC using the surface plasmon resonance (SPR) of the peptide complex of the present invention. The upper graphs show the results for the peptide complex with dimer structure No. 48 in Table 3, and the lower graphs show the results for the peptide complex with dimer structure No. 56 in Table 3.

### DETAILED DESCRIPTION OF THE INVENTION

Hereinafter, the present invention is described in detail with reference to the drawings. The present invention is not limited to the description below and includes modifications made as appropriate based on the description below within a scope obvious to those skilled in the art.

The first invention in this specification relates to a peptide complex. The peptide complex comprises a first peptide and has TrkB binding activity. The peptide complex also has TrkB agonist activity.

### Peptide complex

The peptide complex is a peptide comprising a first peptide and another peptide or a compound, a peptide-containing compound, or a pharmaceutically acceptable salt thereof. The peptide complex may contain 1, 2 or more (3 or more) first peptides. The peptide complex may further contain 1 or more partial peptides different from the first peptide. In the peptide complex, the first peptide and the partial peptide are preferably bonded via a linker. The peptide complex may be a homomultimer comprising only peptides having the same amino acid sequence. The peptide complex may be a heteromultimer comprising peptides having different amino acid sequences. The peptide complex is preferably a homodimer having two first peptides having the same amino acid sequence, which are bonded to each other via a linker. The peptide complex may have TrkB binding activity. The first peptide and the partial peptide comprising the peptide complex preferably have TrkB binding activity. As shown in the Examples, the first peptide forms a peptide complex structure via a linker, thereby exhibiting TrkB agonist activity.

The peptide complex may consist of a first peptide, a second peptide, and a linker connecting the first peptide and the second peptide. In this case, the second peptide may be identical to or different from the first peptide, and preferably has an amino acid sequence represented by SEQ ID NO: 1 or consists of an amino acid sequence with substitution, deletion, addition, or insertion of 1 to 4 amino acids in the amino acid sequence represented by SEQ ID NO: 1. The homology between the first peptide and the second peptide is preferably from 80% to 100% inclusive. The first peptide and the second peptide are preferably homodimers which are substantially the same.

### First peptide

The first peptide has an amino acid sequence represented by X¹-X²-X³-X⁴-3Py-W-X⁵-X⁶-X⁷-X⁸-X⁹-X¹⁰-V-X¹¹-C (SEQ ID NO: 1) or consists of an amino acid sequence with substitution, deletion, addition, or insertion of 1 to 4 (1, 2, 3, or 4) amino acids in the amino acid sequence represented by SEQ ID NO: 1. The substituted amino acid includes a pharmaceutically acceptable salt of the amino acid. A preferred example of the peptide having a sequence consisting of an amino acid sequence with substitution, deletion, addition, or insertion of amino acids in the amino acid sequence represented by SEQ ID NO: 1 is a peptide having a sequence with conservative amino acid substitution in the amino acid sequence represented by SEQ ID NO: 1.

### Conservative amino acid substitution

When 1, 2, or 3 amino-acid residues are substituted, deleted, added, or inserted from a particular sequence, conservative amino acid substitutions are preferably performed. "Conservative amino acid substitution" means substitution with a functionally equivalent or similar amino acid. Conservative amino acid substitution in a peptide causes static changes in the amino acid sequence of the peptide. For example, 1 or more amino acids having similar polarities act in a functionally equivalent manner to cause a static change in the amino acid sequence of the peptide. In general, substitution within the group can be considered conservative in structure and function. However, as is obvious to those skilled in the art, the role played by a particular amino-acid residue can be determined by its implications in the three-dimensional structure of the molecule comprising that amino acid. For example, a cysteine residue can take a less polar, oxidized (disulfide) form compared to the reduced (thiol) form. The long aliphatic moiety of an arginine side chain can constitute a structurally and functionally important feature. A side chain comprising an aromatic ring (such as tryptophan, tyrosine, and phenylalanine) can also contribute to ion-aromatic or cation-pi interactions. In such a case, the substitution of an amino acid having these side chains with an amino acid belonging to an acidic or nonpolar group can be structurally and functionally conservative. Residues of proline, glycine, cysteine (in disulfide form), and the like can have a direct effect on the steric structure of the main chain and often cannot be substituted without structural distortion.

Conservative amino acid substitution includes specific substitutions based on the side chain similarity (L. Lehninger, Biochemistry, 2nd edition, pp. 73-75, Worth Publishers, New York (1975)) and typical substitutions, as shown below.

Conservative amino acid substitution is preferably, for example, substitution to another amino acid belonging to the same group to which an amino acid belongs in the group of natural amino acids classified based on their common side-chain properties, as follows.

Hydrophobic (also referred to as non-polar) amino acids: Hydrophobic amino acids are amino acids that are hydrophobic (non-polar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

The hydrophobic amino acids can also be further classified into the following groups. Aliphatic amino acids: Aliphatic amino acids are amino acids having a fatty acid or hydrogen in a side chain, and include Ala, Gly, Val, Ile, and Leu.

Aliphatic, branched amino acids: Aliphatic, branched amino acids are amino acids having a branched fatty acid in a side chain and include Val, Ile, and Leu.

Aromatic amino acids: Aromatic amino acids are amino acids having an aromatic ring in a side chain and include Trp, Tyr, and Phe.

Hydrophilic (also referred to as polar) amino acids: Hydrophilic amino acids are amino acids that are hydrophilic (polar) and include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acids can also be further classified into the following groups.

Acidic amino acids: Acidic amino acids are amino acids with an acidic side chain and include Asp and Glu.

Basic amino acids: Basic amino acids are amino acids with a basic side chain and include Lys, Arg, and His.

Neutral amino acids: Neutral amino acids are amino acids with a neutral side chain and include Ser, Thr, Asn, Gln, and Cys.

Gly and Pro can be also classified as "amino acids which affect the direction of the main chain," and amino acids having a sulfur molecule in a side chain, such as Cys and Met, can be classified as "sulfur-containing amino acids."

Herein, "amino acids" include not only natural amino acids but also non-natural amino acids. Examples of non-natural amino acids include N-alkyl amino acids in which the natural amino acids above are N-alkylated, and amino acids in which nitrogen that forms a peptide bond is modified with a branched or unbranched lower (e.g., C1 to C5, preferably C1 to C3, or more preferably C1) alkyl group. Among the N-alkyl amino acids, N-ethyl amino acids, N-butyl amino acids, and N-methyl amino acids are preferable, and N-methyl amino acids are more preferable. Non-natural amino acids include chemically modified amino acids such as D-amino acids, β-amino acids, γ-amino acids, amino acid variants, and amino acid derivatives; and amino acids which are not constituent materials of proteins in vivo such as norleucine and ornithine. Non-natural amino acids further include amino acids in which a functional group is further added to a side chain of a natural amino acid or is substituted with another functional group (such as an amino acid with substitutions or additions in an arylene group moiety or alkylene group moiety of a side chain, an amino acid with increased carbon atoms in the arylene group, alkylene group, or alkyl group of a side chain, an amino acid with substitutions in an aromatic ring of a side chain, or a heterocyclized or condensed and cyclized amino acid).

The addition or substitution of a functional group or other structure to a side chain of a natural amino acid can impart properties different from those of the natural amino acid. For example, A4p is alanine with a piperidyl group attached to a side chain, wherein the addition of the piperidyl group makes A4p basic and polar, unlike alanine, which belongs to the non-polar amino acid group.

In other words, a non-natural amino acid having similar side-chain properties can be included in the group of natural amino acids above based on their common side-chain properties. For example, N-methylarginine (MeR), which is an N-methylated amino acid of arginine belonging to the basic amino acid group, is a non-natural amino acid but can be classified as a basic amino acid because it exhibits basic properties. Thus, non-natural amino acids that exhibit similar side-chain properties to amino acids can also be included as targets for conservative amino acid substitution.

Non-limiting examples of non-natural amino acids include N-methyl amino acids, 4Py, alT, Cit, P4Sh, F4CON, F4COO, 3Py, HyP, SMe, A4paa, Atp, Hgl, KAc, Nal1, W6N, W7N, and PeG. N-methyl amino acids can be classified into N-alkyl amino acids or classified according to the side-chain properties of their non-N-methylated original amino acid.

### Peptide length

The first peptide may include, for example, the amino acid sequences represented by SEQ ID NOs: 2 to 41 or an amino acid sequence of positions 1 to 15 of the amino acid sequence and an added amino-acid residue. The added amino-acid residue may be included in a peptide forming a cyclic structure or may be further added in a linker-like manner from the cyclic peptide. Although the number of amide bonds (the number and length of the amino acids) in the peptide and the peptide moiety is not particularly limited, the total number of amino-acid residues (referring to the number of amino-acid residues included in the peptide forming a cyclic structure; when amino-acid residues are added in a linker-line manner from the cyclic peptide, these amino acids are not included) is preferably within 20 residues. The peptide length, in terms of amino-acid residues, is preferably 6 or more, 7 or more, 8 or more, 9 or more, 10 or more, or 11 or more and preferably 19 or less or 18 or less. More preferably, the peptide length, in terms of amino-acid residues, is from 15 to 17 inclusive, and most preferably, the number of amino acids is 16.

In a preferred example of the first peptide,
X¹ is an amino acid having an aromatic ring in a side chain,
X² is any amino acid,
X³ is a secondary amino acid,
X⁴ is an amino acid having an aromatic ring in a side chain, wherein the aromatic ring may be substituted,
X⁵ is a secondary amino acid,
X⁶ is an amino acid having a chain alkyl group in a side chain,
X⁷ is an amino acid having an aromatic ring in a side chain, wherein the aromatic ring may be substituted,
X⁸ is an amino acid having a chain alkyl group in a side chain,
X⁹ is any amino acid,
X¹⁰ is an amino acid having an aromatic ring in a side chain, and
X¹¹ is any amino acid.

In a preferred example of the first peptide,
X² is an amino acid having a chain or cyclic alkyl group in a side chain,
X³ is proline or a proline derivative,
X⁴ is tyrosine or an amino acid in which a hydroxyl group of tyrosine is substituted with another functional group,
X⁵ is proline or a proline derivative,
X⁶ is an amino acid having a chain alkyl group and a polar group in a side chain,
X⁷ is tyrosine or an amino acid in which a hydroxyl group of tyrosine is substituted with another functional group,
X⁸ is an amino acid having a chain alkyl group and a polar group in a side chain, and
X⁹ is an amino acid having a functional group with 3 or more carbon atoms in a side chain.

In a preferred example of the first peptide,
X² is an amino acid further having a polar group in a side chain (an amino acid having a chain or cyclic alkyl group and a polar group in a side chain),
X⁶ is an amino acid having a chain alkyl group and a hydroxyl group in a side chain,
X⁷ is tyrosine or an amino acid in which a hydroxyl group of tyrosine is substituted with another functional group,
X⁸ is an amino acid having a chain alkyl group and a hydroxyl group which may be substituted in a side chain, and
X⁹ is an amino acid having a chain or cyclic alkyl group in a side chain.

Amino acid having an aromatic ring in a side chain, wherein the aromatic ring may be substituted

An "amino acid having an aromatic ring in a side chain, wherein the aromatic ring may be substituted" is an amino acid having an aromatic ring in a side chain, wherein, for example, the aromatic ring is a phenyl group or an indole ring, and some Cs thereof may be substituted with other atoms, such as N. The aromatic ring may be a heterocycle. The amino acid may be, for example, an amino acid in which a hydroxyl group of tyrosine is substituted with another functional group. In one aspect, an "amino acid having an aromatic ring in a side chain, wherein the aromatic ring may be substituted" is preferably an amino acid having a condensed ring in a side chain. For example, the amino acid may be an amino acid having an indole ring or a naphthalene structure, and some Cs thereof may be substituted with other atoms such as N. The substituent is not particularly limited, and can be optionally selected from an alkyl group, a cycloalkyl group, a hydroxyl group, a halogen, and the like. Examples of the "amino acid having an aromatic ring in a side chain, wherein the aromatic ring may be substituted" include tyrosine, tryptophan, histidine, and phenylalanine. The amino acid may be, for example, a non-natural amino acid such as 4Py, F4CON, F4COO, or 3Py.

The "amino acid having an aromatic ring in a side chain, wherein the aromatic ring may be substituted" may be an "amino acid having an aromatic ring in a side chain."

### Amino acid having a chain or cyclic alkyl group in a side chain

The "amino acid having a chain or cyclic alkyl group in a side chain" is an amino acid having an alkyl group in a side chain, such as lysine, alanine, proline, glycine, or leucine, wherein the side chains of these amino acids may or may not be substituted and the alkyl group may be a chain or cyclic alkyl group.

The "amino acid having a chain alkyl group in a side chain" is an amino acid having a chain alkyl group in a side chain.

### Amino acid having a polar group in a side chain

The "amino acid having a polar group in a side chain" is an amino acid having a polar group in a side chain, such as tyrosine, lysine, arginine, glutamic acid, glutamine, serine, and threonine. Non-limiting examples of the polar group to be bonded include a hydroxyl group, a urea group, a carboxylic acid, an amide group, and an ether.

### Amino acid in which a hydroxyl group of tyrosine is substituted with another functional group

The "amino acid in which a hydroxyl group of tyrosine may be substituted with another functional group" may be an amino acid in which a hydroxyl group of tyrosine is substituted with another polar group, non-limiting examples of which include a urea group, a carboxylic acid, an amide group, and an ether. Non-limiting examples of the amino acid include F4CON, F4COO, and Y

### Secondary amino acid

"Secondary amino acid" means an amino acid having a secondary amine (amino acid with two groups other than hydrogen attached to the amino group). Examples of the secondary amino acid include proline and proline derivatives. Examples of proline derivatives include HyP and P4Sh.

### Amino acid having a chain alkyl group and a polar group in a side chain

The chain alkyl group and the polar group in the amino acid having a chain alkyl group and a polar group in a side chain are as described above. An example of the polar group is a hydroxyl group. Examples of the amino acid having a chain alkyl group and a polar group in a side chain include S and SMe.

### Amino acid having a chain or cyclic alkyl group and a polar group in a side chain

The chain or cyclic alkyl group and the polar group in the amino acid having a chain or cyclic alkyl group and a polar group in a side chain are as described above. Examples of the amino acid having a chain or cyclic alkyl group and a polar group in a side chain include alT, Cit, D, E, L, Q, and S.

### Amino acid having a functional group with three or more carbon atoms in a side chain

The "amino acid having a functional group with three or more carbon atoms in a side chain" means an amino acid having 1 or more functional groups in a side chain with three or more total carbon atoms in the functional groups. Examples of the amino acid having a functional group with three or more carbon atoms in a side chain include A4paa, Atp, E, Hgl, KAc, and L.

In a preferred example of the first peptide,
X¹ is 3-(4-pyridyl)-L-alanine (4Py) or F,
X² is allo-threonine (alT), L-citrulline (Cit), D, E, L, Q, or S,
X³ is P or cis-4-hydroxy-L-proline (P4Sh),
X⁴ is L-4-carbamoylphenylalanine (F4CON), 4-carboxyl-L-phenylalanine (F4COO), or Y,
X⁵ is hydroxyproline (HyP), P, or cis-4-hydroxy-L-proline (P4Sh),
X⁶ is S or T,
X⁷ is L-4-carbamoylphenylalanine (F4CON) or Y,
X⁸ is S or (S)-2-amino-3-methoxypropanoic acid (SMe),
X⁹ is (S)-2-amino-3-(1-(carboxymethyl)piperidin-4-yl)propanoic acid (A4paa), (2S)-2-amino-3-(oxan-4-yl)propanoic acid (Atp), E, L-2-aminoadipic acid (Hgl), N6-acetyl-L-lysine (KAc), or L,
X¹⁰ is 3-(3-pyridyl)-L-alanine (3Py) or 3-(4-pyridyl)-L-alanine (4Py),
X¹¹ is A, E, N-methyl-glutamic acid (MeE), P, S, or W.

A preferred example of the first peptide is a peptide having an amino acid sequence represented by F-L-P-Y-3Py-W-P-T-Y-S-L-3Py-V-W-C (SEQ ID NO: 2) ("3py" means 3-(3-pyridyl)-L-alanine) or a peptide consisting of an amino acid sequence with substitution, deletion, addition, or insertion of 1 to 4 amino acids in the amino acid sequence represented by SEQ ID NO: 2. When an amino acid is substituted or deleted, the position of the amino acid is preferably any of the first, second, third, fourth, seventh, eighth, ninth, tenth, eleventh, twelfth, or fourteenth position in the amino acid sequence of SEQ ID NO: 2.

A preferred example of the first peptide has an amino acid sequence with a further addition of glycine at the C-terminus.

A preferred example of the first peptide is a peptide consisting of an amino acid sequence represented by any one of SEQ ID NOs: 2 to 41 or a pharmaceutically acceptable salt thereof. Examples of the pharmaceutically acceptable salt include inorganic acid salts, organic acid salts, inorganic base salts, organic base salts, and acidic or basic amino acid salts. Examples of the inorganic acid salts include hydrochlorides, hydrobromides, sulfates, nitrates, and phosphates. Examples of the organic acid salts include acetates, succinates, fumarates, maleates, tartrates, citrates, lactates, stearates, benzoates, methanesulfonates, and p-toluenesulfonates. Examples of the inorganic base salts include alkali metal salts such as sodium salts and potassium salts; alkali earth metal salts such as calcium salts and magnesium salts; aluminum salts; and ammonium salts. Examples of the organic base salts include diethylamine salts, diethanolamine salts, meglumine salts, and N,N'-dibenzylethylenediamine salts. Examples of the acidic amino acid salts include aspartates and glutamates. Examples of the basic amino acid salts include arginine salts, lysine salts, and ornithine salts. The first peptide and the complex may be pharmaceutically acceptable salts or solvates. An example of the solvate is a hydrate.

A preferred example of the first peptide is a cyclic peptide. A preferred example of the first peptide is a cyclic peptide having a chloroacetylated amino-acid residue as an amino-acid residue at the N-terminus and a cysteine residue in the peptide, wherein the amino-acid residue at the N-terminus and the cysteine residue are bonded to each other.

### Cyclic peptide

The cyclic peptide refers to a peptide wherein two amino acids are bonded to form a ring in whole or in part. In the present application, the cyclic peptide also encompasses peptides wherein the amino acids form a cross-linked structure, peptides wherein a cyclic structure is formed by a lactam ring formation or macrocyclization reaction, and peptides having a lasso peptide-like structure. In other words, in the present application, the cyclic peptide may have a linear chain portion as long as a portion of the peptide forms a cyclic structure.

In the present specification, some amino acids may be modified for the cyclization of the peptide. The peptide also encompasses peptides comprising such modified amino acids. An example of a modification for cyclization is the addition of a chloroacetyl group to an amino acid at the N-terminus, which binds to a cysteine residue in the peptide to form a ring. The peptide of the present application also encompasses peptides comprising various (natural/non-natural) amino acids with added chloroacetyl groups.

Peptides generally have poor metabolic stability in vivo and are large in size, which makes it difficult for the peptides to permeate cell membranes. In order to address this problem, a method that cyclizes the peptides has been used. It has been suggested that the cyclization of the peptides improves protease resistance, improves metabolic stability, and limits conformational changes, thereby increasing rigidity and improving membrane permeability and affinity with target proteins.

The cyclization of the peptides can be performed according to known methods. Although not limited to this, for example, by designing the peptide such that it comprises two or more cysteine residues, a cyclic structure can be formed by a disulfide bond after translation. The cyclization can also be achieved by synthesizing a peptide having a chloroacetyl group at the N-terminus and positioning cysteine residues in the peptide using a genetic code reprogramming technique according to the method of Goto et al (Y Goto, et al. ACS Chem. Biol. 3 120-129 (2008)). This causes the mercapto group to do a spontaneous nucleophilic attack on the chloroacetyl group after translation, and the peptide is cyclized by a thioether bond. Other combinations of amino acids that bond to form rings may be positioned within the peptide to achieve cyclization by the genetic code reprogramming technique. Alternatively, a peptide having a cyclamide at the N-terminus may be synthesized and cyclized by positioning an L-2-aminoadipic acid residue in the peptide and creating a bond between them. Thus, the cyclization method is not particularly limited and any known cyclization method can be used.

### Linker

The first peptide or partial peptide may have an added linker. The linker may have a structure in which a plurality of peptide-containing compounds are linked to each other in the peptide-containing complex. Examples of the linker include an amino acid linker (a peptide linker), a chemical linker, a fatty acid linker, a nucleic acid linker, and a glycan linker, and the linker may be a complex of the chemical linker, the peptide linker, and the like. An example of the chemical linker is a polyethylene glycol (PEG) linker. The PEG linker may be a linker consisting of 1 to 24 or 1 to 36 ethylene glycol units. The linker may also be a fatty acid linker comprising a divalent chemical moiety derived from a fatty acid. The amino acid (peptide) linker is a linker comprising at least one amino acid, and, for example, a glycine-rich peptide linker of a peptide such as one having the sequence [Gly-Gly-Gly-Gly-Ser]n (wherein n is 1, 2, 3, 4, 5, or 6), similar to those described in U.S. Patent No. 7,271,149, and the serine-rich peptide linker described in U.S. Patent No. 5,525,491 can be used. Although not limited to the following, the addition of the linker may change the physical properties (e.g., solubility) of the peptide. In addition, a combination of the linkers above may be suitable. For example, Gly-Lys may be bonded as an amino acid linker, and a PEG linker may be bonded to the terminus of the side chain of Lys. The linker may also have a structure wherein the amino acids and PEGs are bonded alternately, such as PEG-amino acid-PEG. A preferred example of the linker is a linker having a structure shown in Table 2.

The linker may be added at any position. For example, the linker may be located on the N-terminus side of the peptide, bonded to Cys, which forms a cyclic structure by binding to the amino acid at the 1st position, or it may be bonded to an amino acid in the cyclic peptide. A preferred example of the linker is a linker bonded to the Cys located on the N-terminus side, or a linker bonded to the side chain of an amino acid in the cyclic peptide.

For example, in the amino acid sequences represented by SEQ ID NOs: 3 to 41, the Gly at the 16th position can be regarded as a linker. Although not limited to the following, for example, in the case of a cyclic peptide wherein the 1st amino acid of the amino acid sequence represented by SEQ ID NOs: 3 to 41 is bonded to the 15th amino acid, Cys, and there are two cyclic peptide structures wherein glycine is further added to the 15th Cys that have a dimer structure via the structure shown in Table 2 from the added glycine, the linker structure can be said to be the structure shown in Table 2 or can be regarded as a structure in which the structure shown in Table 2 is bonded to glycine.

### TrkB

TrkB refers to tropomyosin receptor kinase B. TrkB is also known as tyrosine receptor kinase B, a BDNF/NT-4 receptor, and a high-affinity neurotrophin receptor. Human TrkB is a protein encoded by the NTRK2 gene (Entrez Gene ID: 4915). TrkB is located at the cell membrane and is activated when the ligand binds to the extracellular domain of the receptor. TrkB is a receptor for brain-derived neurotrophic factor (BDNF) and binds to BDNF in a ligand-specific manner. When BDNF, which is a dimer, binds to TrkB, TrkB is dimerized, leading to the autophosphorylation of tyrosine residues by the kinase domain. As a result, three main signal transduction pathways comprising mitogen-activated protein kinase (MAPK), phosphatidylinositol 3-kinase (PI3K), and phospholipase C-γ1 (PLC-γ1) are activated. Several neurotrophins, such as BDNF, neurotrophin-4 (NT-4), and neurotrophin-3 (NT-3), have been reported to activate TrkB, thereby activating a plurality of effects, including neuronal differentiation, neuronal repair, neuronal plasticity, proliferation, and survival. Although not limited to the following, herein, TrkB is human TrkB or non-human animal TrkB, such as mouse TrkB, rat TrkB, or rabbit TrkB, and is preferably human TrkB.

### TrkB binding activity

The peptide complex of the present invention has TrkB binding activity. The peptide complex of this invention is preferably one that binds specifically to TrkB. For example, the peptide complex of this invention preferably does not exhibit TrkA or TrkC binding activity and exhibits TrkB binding activity. TrkA belongs to the same neurotrophin family as TrkB, but unlike TrkB, it binds to NGF (nerve growth factor) instead of BDNF. TrkC belongs to the same neurotrophin family as TrkB, but it binds to neurotrophin-3. Although not limited to the following, the binding state of the peptide of the present invention to TrkB can be expressed using the affinity constant Ka, dissociation constant Kd, association rate constant kon, and dissociation rate constant koff as indicators.

The affinity constant Ka and the dissociation constant Kd are indicators showing the binding affinity or binding strength between two molecules in equilibrium, and the dissociation constant Kd is the reciprocal of the affinity constant Ka. In other words, the smaller the value of the dissociation constant Kd, the stronger the binding. The affinity constant Ka, dissociation constant Kd, association rate constant kon, and dissociation rate constant koff, which show the binding state of the peptide to TrkB (e.g., human TrkB), can be determined using any molecular interaction measurement method known to a person skilled in the art. The binding state of the peptide to TrkB, for example, can be measured by surface plasmon resonance (SPR) spectroscopy. Although not limited to the following, surface plasmon resonance spectroscopy can be performed, for example, using the BIACORE system (BIACORE), which is a biosensor (biomolecular interaction analysis instrument).

One indicator of the TrkB binding activity of the peptide is the dissociation constant Kd. The lower the dissociation constant Kd is, the higher the binding activity (affinity). Although not limited to the following, the dissociation constant Kd of the binding between the peptide and human TrkB is 100 nM or less, 75 nM or less, 50 nM or less, 30 nM or less, 20 nM or less, 15 nM or less, 10 nM or less, 5 nM or less, 1 nM or less, or 100 pM or less. The lower limit of the dissociation constant Kd of the binding between the peptide and human TrkB is not particularly limited.

### TrkB agonist activity

The peptide complex of the present invention preferably has TrkB agonist activity.

TrkB agonist activity refers to the ability to cause effects similar to those caused by BDNF and NT4, which are naturally occurring TrkB agonists. It also refers to the ability to specifically bind to TrkB, dimerize the receptor above, and activate signal transduction pathways.

"TrkB agonist activity" may be evaluated, for example, by a kinase assay system using TrkB as a target protein. For example, although not limited to the following, TrkB agonist activity can be evaluated by an AlphaLISA assay or NFAT assay to detect the phosphorylation of ERK, or a PathHunter assay to identify the effects of a compound on receptor tyrosine kinases by measuring chemiluminescence signal levels of the substrate hydrolyzed by β-galactosidase activity. Although not limited to the following, evaluation examples include evaluations using PathHunter (registered trademark) eXpress TrkB Functional Assay kit (DiscoverX), PathHunter (registered trademark) TrkB Functional Assay kit (Cosmo Bio), and CellSensor (registered trademark) TrkB-NFAT-bla CHO-K1 Cell Line (Invitrogen). For any of the evaluation systems, if TrkB agonist activity is detected when the peptide or the peptide complex is used at an optimal concentration under optimal conditions according to standard procedures, it can be said that the peptide or peptide complex has TrkB agonist activity. Although not limited to the following, the agonist activity of the peptide or peptide complex can be calculated based on EC50.

### Nucleic acid

This specification also provides a nucleic acid which encodes the first peptide and the peptide complex. Herein, the "nucleic acid" may be a natural or non-natural nucleic acid, including DNA, RNA, and chimeras thereof, but is not limited thereto. The nucleic acid can be designed and produced by a known method based on the sequences of the first peptide and the peptide complex.

### Peptide-drug conjugate (PDC)

An example of the peptide complex is a complex including, any of the peptides above, a linker bonded to the peptide, and a substance bonded to this linker. The peptide above can bind to TrkB, thus the complex can transport the substance to TrkB.
The substance may be any substance desired by a person skilled in the art as long as it is a substance desired to be transported to TrkB. Examples of the substance include, but are not limited to, the following substances.

Compounds: Includes low-molecular-weight compounds and medium-molecular-weight compounds, and examples thereof include known low-molecular-weight pharmaceutical agents.

Peptides: May be a peptide that binds to a target in the body and exhibits some effect. For example, the peptide may be a cyclic peptide.

RIs: May be any compound labeled with a radioisotope, such as a radioisotope-labeled low- or medium-molecular-weight compound or antibody. Examples of RIs include compounds for PET scans.

Proteins: May be any protein that exhibits a useful function in the body, such as an antibody or enzyme. Examples of proteins include enzymes used in enzyme replacement therapy.

Nucleic acids: May be any nucleic acid such as DNA and RNA. Examples of nucleic acids include nucleic acid pharmaceuticals.

Molecules used in drug delivery systems (DDS): May be a known molecule used in a DDS such as a liposome or micelle. The DDS molecule may further contain a compound such as a pharmaceutical inside.

The substance desired to be transported to TrkB may be a complex of the substances listed above.

### Composition

The composition relates to a composition comprising the peptide complex and a carrier. An example of the carrier is a mixture of one or more of water such as sterile water, pure water, or distilled water; a physiological saline solution; a glucose solution; an alcohol such as ethanol; a polyalcohol such as glycerol, propylene glycol, or polyethylene glycol; a sterile organic solvent; a water-soluble starch; and PBS.

### Composition for a pharmaceutical

The composition for a pharmaceutical (hereinafter, also referred to as "pharmaceutical composition") includes the peptide above or a pharmaceutically acceptable salt or solvate thereof (hereinafter, they are also referred to simply as the "peptide" for simplicity). The pharmaceutical composition preferably includes an effective amount of the peptide above as an active ingredient.

TrkB-related disorders, for which the pharmaceutical is applicable, refer to any disorders caused by, exacerbated by, or otherwise associated with increased or decreased expression or activity of TrkB, or disorders caused or exacerbated by downregulations in the BDNF signaling cascade or any other intracellular signaling cascade activated via TrkB. Examples of such neurological disorders and psychiatric disorders include neurodegenerative disorders (including, but not limited to, Alzheimer's disease and related dementias, Parkinson's disease, Huntington's disease, Lewy body disease and related movement disorders, amyotrophic lateral sclerosis, glaucoma, and Friedreich's ataxia and related spinocerebellar ataxias), depression, anxiety, autism, schizophrenia, post-traumatic stress disorder, CNS disorders, strokes, and traumatic brain injuries. Examples of metabolic disorders include obesity and polyphagia. Cancers or cancerous diseases refer to any medical diseases characterized by the growth, neoplasia, abnormal proliferation, invasion, or metastasis of malignant cells, and examples thereof include both solid tumor cancers and non-solid tumor cancers (hematologic malignancies), such as leukemia. Examples of solid tumors include sarcomas and carcinomas. Sarcomas are nonepithelial tumors in blood vessels, bones, adipose tissues, ligaments, lymphatic vessels, muscles, or tendons, and carcinomas are epithelial tumors in the inner layers of the skin, glands, or organs. Tumors refer to solid masses of neoplasm and/or malignant cells.

The route of administration for the pharmaceutical composition above is not particularly limited, and the pharmaceutical composition may be administered orally or parenterally. Examples of parenteral administration include administration by injection such as by intramuscular injections, intravenous injections, or subcutaneous injections; transdermal administration; and transmucosal (transnasal, oral, ocular, pulmonary, vaginal, or rectal) administration.

The peptides in the pharmaceutical composition can be modified in various ways in view of their metabolic and excretory properties. For example, polyethylene glycol (PEG) or a glycan can be added to polypeptides to increase their retention time in blood and decrease their antigenicity. In addition, biodegradable polymer compounds such as poly(lactic-co-glycolic acid) (PLGA); porous hydroxyapatite; liposomes; surface-modified liposomes; and emulsions, nanoparticles, nanospheres, and the like prepared from unsaturated fatty acids may be used as a sustained release substrate, and the polypeptide may be encapsulated therein. When the pharmaceutical composition is administered transdermally, a weak electric current can be applied to the surface of the skin to permeate the stratum corneum (iontophoresis method).

The active ingredients of the pharmaceutical composition may be used as they are, or a pharmaceutically acceptable carrier, excipient, additive, or the like may be added to the active ingredients to formulate the pharmaceutical composition. Examples of the dosage form include a liquid (e.g., an injection), a dispersant, a suspension, a tablet, a pill, a powder, a suppository, a powdered medicine, fine granules, granules, a capsule, a syrup, a troche, an inhalant, an ointment, eye drops, nose drops, ear drops, and a gel patch.

The formulation may be performed through conventional means using, for example, an excipient, a binder, a disintegrant, a lubricant, a solvent, a solubilizer, a colorant, a flavor or odor masking agent, a stabilizing agent, an emulsifier, a sorbefacient, a surfactant, a pH adjuster, a preservative, an antioxidant, and the like, as appropriate.

Examples of components used for the formulation include, but are not limited to, purified water; a saline solution; a phosphate buffer solution; dextrose; glycerol; pharmaceutically acceptable organic solvents such as ethanol; animal and vegetable oils; lactose; mannitol, glucose, sorbitol, crystalline cellulose, hydroxypropyl cellulose, starch, cornstarch, silicic anhydride, aluminum magnesium silicate, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, and human serum albumin.

The sorbefacient that improves absorption of poorly absorbable drugs may be, for example, a surfactant such as polyoxyethylene lauryl ethers, sodium lauryl sulfate, or saponin; a bile salt such as glycocholate, deoxycholate, or taurocholate; a chelating agent such as EDTA and salicylic acids; fatty acids such as caproic acid, capric acid, lauric acid, oleic acid, linoleic acid, or mixed micelles; enamine derivatives; N-acyl collagen peptides; N-acyl amino acids; cyclodextrins; chitosans; and nitric oxide donors.

The pill or tablet may also be coated with a saccharide or substance for gastric or enteric coating. The injection may contain distilled water for injections, a saline solution, propylene glycol, polyethylene glycol, a vegetable oil, and alcohols. The injection may further contain a humectant, an emulsifier, a dispersant, a stabilizing agent, a solvent, a solubilizer, a preservative, and the like.

The pharmaceutical composition of the present invention may be administered in combination with other pharmaceuticals or treatments useful for the disorders above.

When the pharmaceutical composition of the present invention is administered to mammals (e.g., humans, mice, rats, guinea pigs, rabbits, dogs, horses, monkeys, pigs, sheep, and the like), especially humans, the dose depends on the symptoms, age, sex, weight, and sensitivity differences of the subject, administration method, administration interval, types of active ingredients, and type of formulation. The dose is not particularly limited, but amounts of, for example, 30 µg to 1000 mg, 100 µg to 500 mg, or 100 µg to 100 mg may be administered for 1 or several doses. For administration by injection, 1 µg/kg to 3000 µg/kg or 3 µg/kg to 1000 µg/kg may be administered for 1 or several doses, depending on the weight of the subject.

### Diagnostic composition

The peptide of the present invention binds to TrkB, and thus can be used as a diagnostic agent to detect TrkB. The diagnostic agent may be a detection agent for detecting the expression level of TrkB. When used as a detection agent, the peptide of the present invention may be detectably labeled. Thus, the first peptide, the peptide complex, or a composition comprising them can be used as a diagnostic agent for detecting TrkB.

### Research composition

The peptide of the present invention binds to TrkB, and thus can be used preferably in research involving TrkB.

### Cell culture composition

The cell culture composition is a composition used in cell cultures. The peptide of the present application has TrkB agonist activity, and thus can also be used as a reagent or additive for a cell culture medium, preferably a cell culture medium for culturing mammalian cells, or more preferably a cell culture medium for culturing human cells.

The peptide of the present invention can also be used as a reagent and additive for a medium for preparing transplantable neurons for use in the treatment of neurodegenerative disorders. Although not limited to the following, the transplantable neurons can be prepared by culturing pluripotent stem cells, multipotent stem cells, or progenitor cells. BDNF, which is a naturally-occurring TrkB agonist, is also expressed in non-neuronal tissues such as bones, cartilage, kidneys, and the tooth germ, and is also produced at the cellular level by periodontal ligament cells, osteoblasts, immunocompetent cells, vascular endothelial cells, and epithelial cells. Since BDNF is a signaling molecule that plays a functional role not only in neurons but also in various non-neuronal cells, the peptide of the present application may be used as a reagent and additive for the preparation of various cells, such as the cells above.

The medium is not particularly limited as long as it is a medium for culturing cells or tissues. The medium may be a serum medium, or preferably a serum-free or low-serum medium.

The culture medium additive may be in a solution form or a dried solid form (e.g., solid, powder, or the like). If the culture medium is in the solution form, it may be used as it is as a culture medium, or it may be diluted using a solvent, mixed with the additive above as needed, and then used as a culture medium. Examples of the solvent used for the dilution include water, a buffer solution, a saline solution, and media for culturing various cells and tissues, which may be used alone or in a combination of 2 or more of them.

If the culture medium additive is in the dried solid form, it may be dissolved in a solvent such as water, a buffer solution, a saline solution, and media for culturing various cells and tissues, mixed with the additive above as needed, and then used as a culture medium.

The content of the peptide complex of the present invention in the medium for culturing cells or tissues or in the medium for cells obtained therefrom is, for example, as a final concentration relative to the total amount of the composition or the medium, about 0.01 nmol/L to about 10000 nmol/L, preferably about 0.1 nmol/L to about 1000 nmol/L, more preferably about 0.5 nmol/L to about 1000 nmol/L, or even more preferably about 1 nmol/L to about 100 nmol/L.

### Abbreviations (General)

Å for angstrom (unit);
BSA for bovine serum albumin;
ClAc for chloroacetyl;
DCM for dichloromethane or methylene chloride;
DIPCI for N,N'-diisopropylcarbodiimide;
DIPEA or DIEA for N,N-diisopropylethylamine;
DMSO for dimethyl sulfoxide;
DMF for N,N-dimethylformamide;
DODT for 3,6-dioxa-1,8-octane-dithiol;
DMEM for Dulbecco's Modified Eagle's Medium;
EC50 for 50% effective concentration;
FBS for fetal bovine serum;
Fmoc as 9-fluorenylmethyloxycarbonyl;
Fmoc-Lys(Fmoc)-OH for N²,N⁶-bis(((9H-fluoren-9-yl)methoxy)carbonyl)-L-lysine;
g for gram (unit);
HATU for O-(7-azabenzotriazol-1-yl)-N,N,N',N'-tetramethyluronium hexafluorophosphate;
HPLC for high-performance liquid chromatography;
LC-MS or LC/MS for liquid chromatography-mass spectrometer;
M for molar (unit);
mg for milligram (unit);
mL for milliliter (unit);
mM for millimolar (unit);
MeCN for acetonitrile;
min for minute (unit);
mm for millimeter (unit);
NHS for N-hydroxysuccinimide;
nm for nanometer (unit);
µL for microliter (unit);
OSu for succinimide;
Pbf for 2,2,4,6,7-pentamethyldihydrobenzofuran-5-sulfonyl group;
PEG for polyethylene glycol;
rpm for revolutions per minute (unit);
TFA for trifluoroacetic acid;
TIS for triisopropylsilane; and
Trt or Tr for trityl group.

### Abbreviations (Non-natural amino acids)

Ahp for (S)-2-aminoheptanoic acid (CAS No. 44902-02-5);
Atp for (2S)-2-amino-3-(oxan-4-yl)propanoic acid (CAS No. 1344910-91-3);
A4paa for (S)-2-amino-3-(1-(carboxymethyl)piperazine-4-yl)propanoic acid (Kishida Chemical Co., Ltd.);
Cit for L-citrulline (CAS No. 372-75-8);
cPEG1c for 3,3'-oxydipropionic acid;
cPEG5c for 4,7,10,13,16-pentaoxanonadecanedioic acid;
cPEG9c for 4,7,10,13,16,19,22,25,28-nonaoxahentriacontanedioic acid;
cPEG13c for 4,7,10,13,16,19,22,25,28,31,34,37,40-tridecaoxatritetracontanedioic acid;
cPEG17c for 4,7,10,13,16,19,22,25,28,31,34,37,40,43,46,49,52-heptadecaoxapentapentacontanedioic acid;
df for D-phenylalanine;
dc for D-cysteine;
F4CON for L-4-carbamoylphenylalanine (CAS No. 223593-04-2);
F4COO for 4-carboxyl-L-phenylalanine (CAS No. 126109-42-0);
Hgl for L-2-aminoadipic acid (CAS No. 1118-90-7);
Hty for homo-L-tyrosine (CAS No. 221243-01-2);
KAc for N6-acetyl-L-lysine (CAS No. 92-04-6);
MeF for N-methyl-L-phenylalanine;
MeA for N-methyl-L-alanine;
MeG for N-methylglycine;
NHS-cPEG13c-NHS for bis(2,5-dioxopyrrolidin-1-yl)4,7,10,13,16,19,22,25,28,31,34,37,40-tridecaoxatritetracontanedioate;
NHS-OCOPEG13OCO-NHS for bis(2,5-dioxopyrrolidin-1-yl)(3,6,9,12,15,18,21,24,27,30,33-undecaoxapentatriacontane-1,35-diyl)biscarbonate;
OCOPEG13OCO for 3,6,9,12,15,18,21,24,27,30,33-undecaoxapentatriacontane-1,35-diylbis(bicarbonate);
PEG2c for 3-(2-(2-aminoethoxy)ethoxy)propanoic acid;
PEG4c for 1-amino-3,6,9,12-tetraoxapentadecan-15-oic acid;
PEG6c for 1-amino-3,6,9,12,15,18-hexaoxahenicosan-21-oic acid;
PEG8c for 1-amino-3,6,9,12,15,18,21,24-octaoxaheptacosan-27-oic acid;
PEG12c for 1-amino-3,6,9,12,15,18,21,24,27,30,33,36-dodecaoxanonatriacontan-39-oic acid;
P4Sh for cis-4-hydroxy-L-proline (CAS No. 618-27-9);
SMe for (S)-2-amino-3-methoxypropanoic acid (CAS No. 32620-11-4);
Tic for L-1,2,3,4-tetrahydroisoquinoline-3-carboxylic acid (CAS No. 74163-81-8);
3Py for 3-(3-pyridyl)-L-alanine (CAS No. 64090-98-8);
4Py for 3-(4-pyridyl)-L-alanine (CAS No. 37535-49-2);
alT for allo-threonine;
HyP for hydroxyproline; and
MeE for N-methyl-glutamic acid.

### EXAMPLES

The present invention is described in more detail below with reference to the Examples. However, the present invention is not limited to the following Examples. A person skilled in the art may easily modify or change the present invention based on the description of the present specification, and these modifications and changes are included in the technical scope of the present invention.

### Chemical synthesis

All raw materials, building blocks, reagents, acids, bases, solid-phase resins, and solvents used in the chemical syntheses of the following Examples were used as they were commercially available, or they can be synthesized by those skilled in the art using organic chemical techniques. An amino acid comprising a protecting group was used as it was commercially available unless otherwise noted.

The elongation of a peptide chain in the solid-phase resin was performed by using the resins described in the Examples as starting materials and using commonly used peptide coupling reaction conditions and Fmoc removal reaction conditions. The reactions were performed using Syro I or Syro II available from Biotage, or Liberty Blue, Liberty Blue HT12, or Liberty Prime available from CEM, which is an automated peptide synthesizer, according to the manufacturer's manual.

The resin used was NovaPEG Rink Amide resin or Seiber Amide resin, and the amount used ranged from 5 mg to 2 g for each peptide.

The amount of the reagent cocktail used for the deprotection of the side chain and the cleavage of the side chain from the solid-phase resin is 4 mL to 50 mL for each peptide, and solutions of the following compositions were used.
A: TFA/H₂O/TIS/DODT (92.5/2.5/2.5/2.5)
B: TFA/H₂O/TIS/DODT (90/2.5/2.5/5)

Common amino acids that were used are listed below, and the side chain protecting groups are indicated in parentheses:
Fmoc-Ala-OH;
Fmoc-Arg(Pbf)-OH;
Fmoc-Asn(Trt)-OH;
Fmoc-Asp(OMpe)-OH;
Fmoc-Cys(Trt)-OH;
Fmoc-Gln(Trt)-OH;
Fmoc-Glu(OtBu)-OH;
Fmoc-Gly-OH;
Fmoc-Leu-OH;
Fmoc-Lys(Boc)-OH;
Fmoc-Lys(Fmoc)-OH;
Fmoc-Phe-OH;
Fmoc-Pro-OH;
Fmoc-Ser(tBu)-OH;
Fmoc-Ser(Trt)-OH;
Fmoc-Thr(tBu)-OH;
Fmoc-Trp(Boc)-OH;
Fmoc-Tyr(tBu)-OH; and
Fmoc-Val-OH.

For purification of the obtained crude peptide, any of the following preparative reversed phase purification devices a)/b)/c)/d) were used unless otherwise noted:
a): Shimadzu prep-HPLC system (LC-20AP, SPD-M20A, CTO-20AC, and CBM-20A);
b): Waters AutoPurification System;
c): Waters AutoPurification System with SQD; or
d): Waters Preparative HPLC System.

The purification conditions, including the columns used, are shown in each Example.

The structure of the chemically synthesized peptides was determined by ESI-MS(+) in mass spectrometry, where the molecular weight was calculated by considering the amino acids used according to the target sequence and the building blocks used as needed. The term "ESI-MS(+)" refers to electrospray ionization mass spectrometry performed in positive ion mode. The detected masses are reported in "m/z" units. Compounds with molecular weights roughly greater than 1,000 were detected as multivalent ions at a high frequency.

For the mass spectrometry of the peptides synthesized in the following Examples, the following basic analyzers and basic conditions 1 or 2 were used unless otherwise noted. The slope B (%) was analyzed using any of the X/Y/Z conditions.

### Basic analyzer and basic conditions 1

Analyzer: Shimadzu LC/MS system (LC-20ADXR, CTO-20AC, SPD-M20A, SIL-20AXR, CBM-20A, and LCMS-2020);
Column: Kinetex EVO C18, 2.6 µm, 2.1 mm × 150 mm, 100 Å;
Column temperature: 60°C;
Mobile phase A: 0.025% TFA in H₂O;
Mobile phase B: 0.025% TFA in MeCN;
Flow rate: 0.5 mL/min;
Wavelength: 225 nm PDA; and
Slope B (%): X: 20% to 60%/7.15 min, 60% to 95%/0.3 min, 95% to 95%/1.55 min; Y: 5% to 45%/7.15 min, 45% to 95%/0.3 min, 95% to 95%/1.55 min.

### Basic analyzer and basic conditions 2

Analyzer: UPLC H-Class with SQD2 mass spectrometer (Waters);
Column: Kinetex EVO C18, 1.7 µm, 2.1 mm × 50 mm, 100 Å;
Column temperature: 60°C;
Mobile phase A: 0.025% TFA in H₂O;
Mobile phase B: 0.025% TFA in MeCN;
Flow rate: 0.6 mL/min;
Wavelength: 220 nm; and
Slope B (%): Z: 5% to 95%/2.10 min, 95% to 95%/0.75 min.

### Example 1: Synthesis of peptide complex (dimer structure No. 2)

In the present example, a peptide complex having the following structure (a complex of peptide sequence No. 3 and linker structure No. 2; dimer structure No. 2 in Table 3) was synthesized. The peptide complex can also be synthesized by the method of Example 1-1 or 1-2.

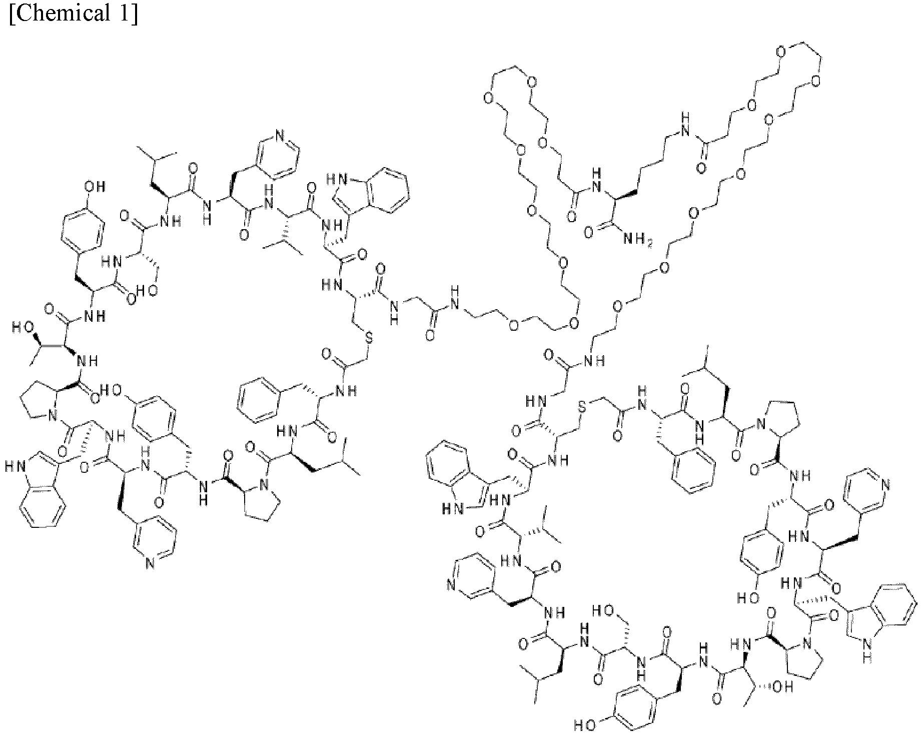

### Example 1-1

A target peptide was synthesized using NovaPEG Rink amide resin (Merck, 0.49 mmol/g), starting with the removal of the Fmoc group by the general method described above. The synthesis was performed using Syro II available from Biotage as a solid-phase synthesizer, according to the manufacturer's manual. For the introduction of each residue, Fmoc-AA/HATU/DIEA (12.6 equivalents/11.2 equivalents/25.2 equivalents per equivalent of resin) was used, and the reaction was performed twice at 25°C for 60 minutes, except that, for the introduction of the linker moiety PEG8c, the reaction was performed once at 25°C for 60 minutes.

For the Fmoc removal, Fmoc was reacted with a 20% piperidine solution in DMF at 25°C for 5 minutes, then the solution was removed, and a 20% piperidine solution in DMF was added again and reacted at 25°C for 15 minutes.

The introduction of a chloroacetyl group into the solid-phase resin containing the Fmoc-protected peptide obtained in the previous step was performed by removing the Fmoc group of the α-amino group by the method described above, and then adding a chloroacetic acid solution in DMF (12.6 equivalents), HCTU solution in DMF (12 equivalents), and DIEA solution in DMF (25 equivalents) to the solid-phase resin and shaking the resultant mixture at room temperature for 30 minutes.

The deprotection of the side chain and the cleavage of the side chain from the solid-phase resin were performed as follows. First, the resin obtained after the chloroacetyl group introduction step was washed 5 times with DMF and 3 times with methylene chloride, and then dried under reduced pressure. A reagent cocktail-A (a mixture of TFA/H₂O/TIS/DODT in a volume ratio of 92.5/2.5/2.5/2.5) was then added to the reaction container containing the solid-phase resin and the resultant mixture was shaken at room temperature for 60 minutes. A reaction solution was collected from a frit by filtration. When the filtrate was added to an excess amount of cooled diisopropyl ether, a white precipitate was generated. The mixture was centrifuged, and the solution was decanted. The resultant solid was washed again with a small amount of a cooled diethyl ether/hexane (1/1) solvent mixture and dried under reduced pressure. The resultant solid was used in the next cyclization reaction.

The cyclization reaction of the peptide was performed by dissolving the peptide in DMSO such that the final concentration of the peptide reached 2.5 mM based on the number of moles of the solid-phase resin, then adding triethylamine (10 equivalents), and shaking the mixture at room temperature overnight. The resultant reaction solution was concentrated under reduced pressure using a Genevac EZ-2 Elite such that the peptide concentration was 25 mM.

The resultant reaction solution above was subjected to solid-phase extraction using a column available from Gilson (Column: Gilson ASPEC C18, 50 mg, 1 mL). (1) The column was washed with an extraction liquid A (0.1% TFA in 95% MeCN/H₂O, 0.3 mL). (2) The column was equilibrated with an extraction liquid B (0.1% TFA in 5% MeCN/H₂O, 0.3 mL). (3) 0.02 mL of the reaction solution above was loaded into the column. (4) The column was washed with the extraction liquid B (0.4 mL). (5) The extraction was performed with the extraction liquid A (0.4 mL). The resultant extract was concentrated under reduced pressure using the EZ-2 Elite.

The purity of one of the main peaks of the target product calculated from an area ratio in an LC/MS (UV wavelength: 220 nm) chromatogram under the following analysis conditions was 55.3%.

Analysis conditions: Retention time = 1.52 min; Column: Kinetex EVO C18, 1.7 µm, 2.1 mm × 50 mm, 100 Å; Mobile phase: A = 0.025% TFA in H₂O, B = 0.025% TFA in MeCN; Temperature: 60°C; Gradient (B conc (%)): 5% to 95% over 2.10 min, then 95% to 95% over 0.75 min; Flow rate: 0.6 mL/min.
ESI-MS(+): Observed m/z value = 1274.4 (M + 4H)⁴⁺.

### Example 1-2

A target peptide was synthesized using NovaPEG Rink amide resin (Merck, 0.49 mmol/g), starting with the removal of the Fmoc group by the general method described above. The synthesis was performed using Syro I available from Biotage as a solid-phase synthesizer, according to the manufacturer's manual. For the introduction of each residue, Fmoc-AA/HATU/DIEA (8.4 equivalents/8 equivalents/16 equivalents per equivalent of resin) was used, and the reaction was performed twice in DMF at 75°C for 20 minutes. However, for the introduction of the 15th residue, the reaction was performed once at room temperature for 20 minutes. For the introduction of the 16th residue, the linker moiety PEG8c, and K, the reaction was performed once at 75°C for 20 minutes.

For the Fmoc removal, Fmoc was reacted with a 20% piperidine solution in DMF at room temperature for 5 minutes, then the solution was removed, and a 20% piperidine solution in DMF was added again and reacted at room temperature for 15 minutes.

The introduction of a chloroacetyl group into the solid-phase resin containing the Fmoc-protected peptide obtained in the previous step was performed by removing the Fmoc group of the α-amino group by the method described above, and then adding a ClAcOSu solution in DCM/DMF, prepared by stirring chloroacetic acid (10.6 equivalents), DIPCI (10.5 equivalents), and HOSu (10.6 equivalents) in DCM and adding the same amount of DMF as DCM, to the solid-phase resin and shaking the resultant mixture at room temperature for 150 minutes.

The deprotection of the side chain and the cleavage of the side chain from the solid-phase resin were performed as follows. First, the resin obtained after the chloroacetyl group introduction step was washed 5 times with DMF and 3 times with methylene chloride, and then dried under reduced pressure. A reagent cocktail-A (a mixture of TFA/H₂O/TIS/DODT in a volume ratio of 92.5/2.5/2.5/2.5) was then added to the reaction container containing the solid-phase resin and the resultant mixture was shaken at room temperature for 90 minutes. A reaction solution was collected from a frit by filtration. When the filtrate was added to an excess amount of cooled diisopropyl ether, a white precipitate was generated. The mixture was centrifuged, and the solution was decanted. The resultant solid was washed again with a small amount of cooled diethyl ether and dried under reduced pressure. The resultant solid was used in the next cyclization reaction.

The cyclization reaction of the peptide was performed by dissolving the peptide in DMSO/H₂O (9/1) such that the final concentration of the peptide reached 1 mM based on the number of moles of the solid-phase resin, then adding triethylamine (10 equivalents), and stirring the mixture at room temperature for 3 hours. Acetic acid was added to the resultant reaction solution, which was then concentrated under reduced pressure using a Genevac HT-12.

The resultant crude product was purified under the following conditions. (Column: Waters XBridge (registered trademark) C18, 50 mm × 150 mm; Mobile phase: A= 0.1% TFA in H₂O, B = 0.1% TFA in MeCN; Temperature: 40°C; Gradient (B conc (%)): 12% to 37% over 3 min, then 37% to 42% over 8 min, then 42% to 60% over 1 min; Flow rate: 120 mL/min.)

The purity of the target product calculated from an area ratio in an LC/MS (UV wavelength: 225 nm) chromatogram under the following analysis conditions was 85.03%.

Analysis conditions: Retention time = 4.82 min; Column: Kinetex EVO C18, 2.6 µm, 2.1 mm × 150 mm, 100 Å; Mobile phase: A= 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min.

ESI-MS(+): Observed m/z value = 1274.1 (M + 4H)⁴⁺.

### Example 2: Synthesis of peptide complex (dimer structure No. 48)

In the present example, a peptide complex having the following structure (a complex of peptide sequence No. 15 and linker structure No. 7; dimer structure No. 48 in Table 3) was synthesized.

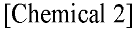

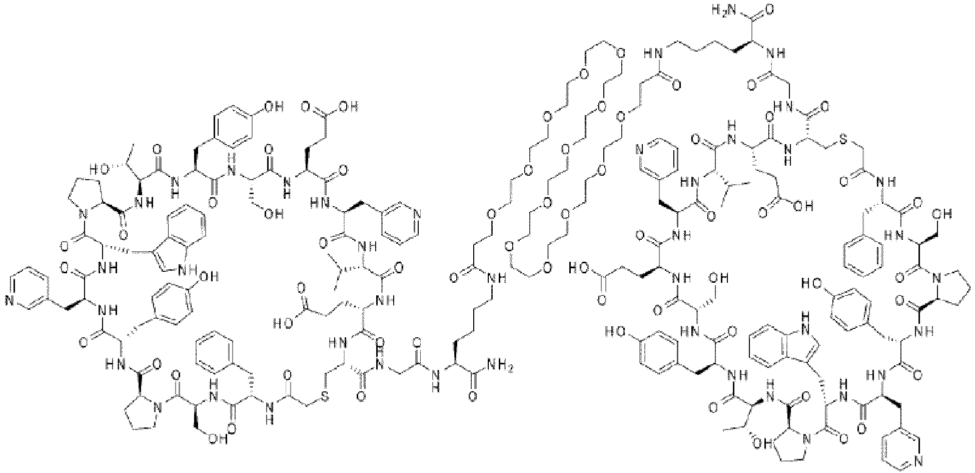

A target peptide was synthesized using Sieber amide resin (Watanabe Chemical Industries, 0.57 mmol/g), starting with the removal of the Fmoc group by the general method described above. The synthesis was performed using Liberty Blue available from CEM as a solid-phase synthesizer, according to the manufacturer's manual. For the introduction of each residue, Fmoc-AA/DIPCI/Oxyma pure (4.2 equivalents/8 equivalents/4 equivalents per equivalent of resin) was used, and the reaction was performed once in DMF at 75°C for 10 minutes. However, for the introduction of the 2nd residue, 3rd residue, 6th residue, and 7th residue, the reaction was performed twice at 75°C for 10 minutes. For the introduction of the 15th residue, the reaction was performed once at 50°C for 20 minutes. For the Fmoc removal, the conditions of a reaction with a 20% piperidine solution in DMF performed at 75°C for 3 minutes were basically used. For the introduction of the 1st residue and 5th residue, the conditions of a reaction with a 20% piperidine solution in DMF performed at room temperature for 5 minutes, removal of the solution, and reaction with a 20% piperidine solution in DMF performed again at room temperature for 10 minutes were used.

The introduction of a chloroacetyl group into the solid-phase resin containing the Fmoc-protected peptide obtained in the previous step was performed by removing the Fmoc group of the α-amino group by the method described above, and then adding a 0.2M chloroacetic acid solution in DMF (5 equivalents), 0.5M HATU solution in DMF (5 equivalents), and 1M DIEA solution in DMF (10 equivalents) to the solid-phase resin and shaking the resultant mixture at room temperature for 30 minutes.

The deprotection of the side chain and the cleavage of the side chain from the solid-phase resin were performed as follows. First, the resin obtained after the chloroacetyl group introduction step was washed 5 times with DMF and 3 times with methylene chloride, and then dried under reduced pressure. A reagent cocktail-A (a mixture of TFA/H₂O/TIS/DODT in a volume ratio of 92.5/2.5/2.5/2.5) was then added to the reaction container containing the solid-phase resin and the resultant mixture was shaken at room temperature for 60 minutes. A reaction solution was collected from a frit by filtration. The solid-phase resin remaining in the reaction container was shaken again with a cleavage cocktail, and the solution component was collected from the frit and mixed with the filtrate described above. When the filtrate was added to an excess amount of a cooled diethyl ether/hexane (1/1) solvent mixture, a white precipitate was generated. The mixture was centrifuged, and the solution was decanted. The resultant solid was washed again with a small amount of cooled diethyl ether and dried under reduced pressure. The resultant solid was used in the next cyclization reaction. The cyclization reaction of the peptide was performed by dissolving the peptide in MeCN/H₂O (1/1) such that the final concentration of the peptide reached 5 mM based on the number of moles of the solid-phase resin, then adding triethylamine (10 equivalents), and shaking the mixture at room temperature for 6 hours. The resultant reaction solution was concentrated under reduced pressure using a Genevac HT-12.

The resultant crude product was purified under the following conditions. (Column: Waters XBridge (registered trademark) C18, 50 mm × 150 mm; Mobile phase: A= 0.1% TFA in H₂O, B = 0.1% TFA in MeCN; Temperature: 40°C; Gradient (B conc (%)): 5% over 2 min, then 5% to 19% over 1 min, then 19% to 24% over 8 min, and then 24% to 60% over 1 min; Flow rate: 1 min (20 mL/min), then 1 min (20 mL/min to 120 mL/min), and then (120 mL/min).)

The resultant cyclic peptide was used as peptide A, and the purity of the cyclic peptide calculated from an area ratio in an LC/MS (UV wavelength: 225 nm) chromatogram under the following analysis conditions was 99.43%.

Analysis conditions: Retention time = 4.52 min; Column: Kinetex EVO C18, 2.6 µm, 2.1 mm × 150 mm, 100 Å; Mobile phase: A= 0.025% TFA in H₂O, B = 0.025% TFA in MeCN; Temperature: 60°C; Gradient (B conc (%)): 5% to 45% over 7.15 min, then 45% to 95% over 0.30 min, and then 95% to 95% over 1.55 min; Flow rate: 0.5 mL/min.

ESI-MS(+): Observed m/z value = 710.4 (M + 3H)³⁺.

The synthesis of the peptide complex was performed by dissolving the peptide A obtained above in DMF such that the final concentration of the peptide complex reached 25 mM, then adding 10 equivalents of triethylamine, then adding 0.5 equivalents of NHS-cPEG13c-NHS at 0°C, and shaking the mixture at room temperature for 1 hour.

The resultant crude product was purified under the following conditions. (Column: Waters XBridge (registered trademark) C18, 30 mm × 150 mm; Mobile phase: A= 0.1% TFA in H₂O, B = 0.1% TFA in MeCN; Temperature: 50°C; Gradient (B conc (%)): 5% to 26% over 3 min, then 26% to 31% over 8 min, and then 31% to 60% over 1 min; Flow rate: 45 mL/min.)

The purity of the target product calculated from an area ratio in an LC/MS (UV wavelength: 225 nm) chromatogram under the following analysis conditions was 93.88%.

Analysis conditions: Retention time = 6.02 min; Column: Kinetex EVO C18, 2.6 µm, 2.1 mm × 150 mm, 100 Å; Mobile phase: A= 0.025% TFA in H₂O, B = 0.025% TFA in MeCN; Temperature: 60°C; Gradient (B conc (%)): 5% to 45% over 7.15 min, then 45% to 95% over 0.30 min, and then 95% to 95% over 1.55 min; Flow rate: 0.5 mL/min.

ESI-MS(+): Observed m/z value = 1228.8 (M + 4H)⁴⁺.

### Example 3: Synthesis of peptide complex (dimer structure No. 57)

In the present example, a peptide complex having the following structure (a complex of peptide sequence No. 17 and linker structure No. 14; dimer structure No. 57 in Table 3) was synthesized.

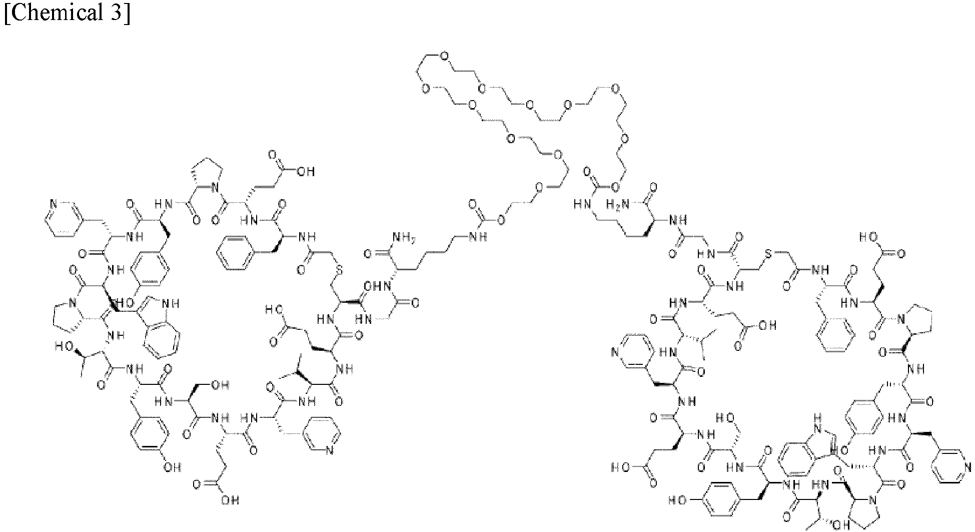

A target peptide was synthesized using Sieber amide resin (Watanabe Chemical Industries, 0.57 mmol/g), starting with the removal of the Fmoc group by the general method described above. The synthesis was performed using Liberty Blue available from CEM as a solid-phase synthesizer, according to the manufacturer's manual. For the introduction of each residue, Fmoc-AA/DIPCI/Oxyma pure (4.2 equivalents/8 equivalents/4 equivalents per equivalent of resin) was used, and the reaction was performed once in DMF at 75°C for 10 minutes. However, for the introduction of the 2nd residue and 6th residue, the reaction was performed twice at 75°C for 10 minutes. For the introduction of the 15th residue, the reaction was performed once at 50°C for 15 minutes or 20 minutes.

For the Fmoc removal, the conditions of a reaction with a 20% piperidine solution in DMF performed at room temperature for 5 minutes, removal of the solution, and reaction with a 20% piperidine solution in DMF performed again at room temperature for 10 minutes, or the condition of a reaction with a 20% piperidine solution in DMF performed at 75°C for 3 minutes were used.

The introduction of a chloroacetyl group into the solid-phase resin containing the Fmoc-protected peptide obtained in the previous step was performed by removing the Fmoc group of the α-amino group by the method described above, and then adding a ClAcOSu solution in DCM/DMF, prepared by stirring chloroacetic acid (5 equivalents), DIPCI (5 equivalents), and HOSu (5 equivalents) in DCM and adding the same amount of DMF as DCM, to the solid-phase resin and shaking the resultant mixture at room temperature for 60 minutes.

The deprotection of the side chain and the cleavage from the solid-phase resin were performed as follows. First, the resin obtained after the chloroacetyl group introduction step was washed 5 times with DMF and 3 times with methylene chloride, and then dried under reduced pressure. A reagent cocktail-B (a mixture of TFA/H₂O/TIS/DODT in a volume ratio of 90/2.5/2.5/5) was then added to the reaction container containing the solid-phase resin and the resultant mixture was shaken at room temperature for 90 minutes. A reaction solution was collected from a frit by filtration. The solid-phase resin remaining in the reaction container was shaken again with a cleavage cocktail, and the solution component was collected from the frit and mixed with the filtrate described above. When the filtrate was added to an excess amount of a diisopropyl ether/hexane (1/1) solvent mixture cooled to 0°C, a white precipitate was generated. The mixture was centrifuged, and the solution was decanted. The resultant solid was washed again with a small amount of diethyl ether cooled to 0°C and dried under reduced pressure. The resultant solid was used in the next cyclization reaction.

The cyclization reaction of the peptide was performed by dissolving the peptide in DMSO/H₂O (9/1) such that the final concentration of the peptide reached 5 mM based on the number of moles of the solid-phase resin, then adding triethylamine (10 equivalents), and shaking the mixture at room temperature for 10 hours. The resultant reaction solution was concentrated under reduced pressure using a Genevac HT-12.

The resultant crude product was purified under the following conditions. (Column: Waters XBridge (registered trademark) C18, 50 mm × 150 mm; Mobile phase: A = 0.1% TFA in H₂O, B = 0.1% TFA in MeCN; Temperature: 40°C; Gradient (B conc (%)): 5% over 2 min, then 5% to 19% over 1 min, then 19% to 24% over 8 min, and then 24% to 60% over 1 min; Flow rate: 1 min (20 mL/min), then 1 min (20 mL/min to 120 mL/min), and then (120 mL/min).)

The resultant cyclic peptide was used as peptide B, and the purity of the cyclic peptide calculated from an area ratio in an LC/MS (UV wavelength: 225 nm) chromatogram under the following analysis conditions was 99.69%.
Analysis conditions: Retention time = 4.51 min; Column: Kinetex EVO C18, 2.6 µm, 2.1 mm × 150 mm, 100 Å; Mobile phase: A = 0.025% TFA in H₂O, B = 0.025% TFA in MeCN; Temperature: 60°C; Gradient (B conc (%)): 5% to 45% over 7.15 min, then 45% to 95% over 0.30 min, and then 95% to 95% over 1.55 min; Flow rate: 0.5 mL/min.

ESI-MS(+): Observed m/z value = 724.4 (M + 3H)³⁺.

The synthesis of the peptide complex was performed by dissolving the peptide B obtained above in DMF such that the final concentration of the peptide complex reached 25 mM, then adding 10 equivalents of triethylamine, then adding 0.5 equivalents of NHS-OCOPEG13OCO-NHS at 0°C, and shaking the mixture at room temperature for 1 hour.

The resultant mixture was purified under the following conditions. (Column: Waters XBridge (registered trademark) C18, 30 mm × 150 mm; Mobile phase: A = 0.1% TFA in H₂O, B = 0.1% TFA in MeCN; Temperature: 50°C; Gradient (B conc (%)): 5% to 26% over 3 min, then 26% to 31% over 8 min, and then 31% to 60% over 1 min; Flow rate: 45 mL/min.)

The purity of the target product calculated from an area ratio in an LC/MS (UV wavelength: 225 nm) chromatogram under the following analysis conditions was 91.04%.

Analysis conditions: Retention time = 5.95 min; Column: Kinetex EVO C18, 2.6 µm, 2.1 mm × 150 mm, 100 Å; Mobile phase: A= 0.025% TFA in H₂O, B = 0.025% TFA in MeCN; Temperature: 60°C; Gradient (B conc (%)): 5% to 45% over 7.15 min, then 45% to 95% over 0.30 min, and then 95% to 95% over 1.55 min; Flow rate: 0.5 mL/min.

ESI-MS(+): Observed m/z value = 1235.7 (M + 4H)⁴⁺.

### Example 4: Synthesis of peptide complex (dimer structure No. 40)

In the present example, a peptide complex having the following structure (a complex of peptide sequence No. 36 and linker structure No. 4; dimer structure No. 40 in Table 3) was synthesized.

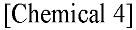

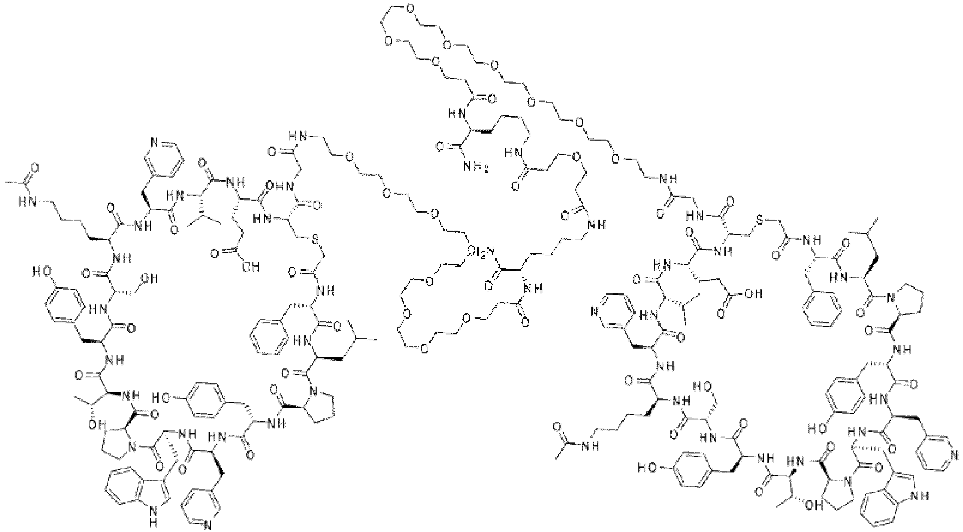

A target peptide was synthesized using Sieber amide resin (Watanabe Chemical Industries, 0.48 mmol/g), starting with the removal of the Fmoc group by the general method described above. The synthesis was performed using Liberty Blue available from CEM as a solid-phase synthesizer, according to the manufacturer's manual. For the introduction of each residue, Fmoc-AA/DIPCI/Oxyma pure (4.2 equivalents/8 equivalents/4 equivalents) was used per equivalent of resin, and the reaction was performed once in DMF at 90°C for 3 minutes. However, for the introduction of the 2nd residue, 3rd residue, 6th residue, and 7th residue, the reaction was performed twice at 90°C for 3 minutes. For the introduction of the 15th residue, the reaction was performed once at 50°C for 15 minutes.

For the Fmoc removal, the conditions of a reaction with a 10% pyrrolidine solution in DMF performed at room temperature for 1 minute, removal of the solution, and reaction with a 10% pyrrolidine solution in DMF performed again at room temperature for 1 minute, or the condition of a reaction with a 10% pyrrolidine solution in DMF performed at 90°C for 1 minute were used.

The introduction of a chloroacetyl group into the solid-phase resin containing the Fmoc-protected peptide obtained in the previous step was performed by removing the Fmoc group of the α-amino group by the method described above, and then adding a 0.2M chloroacetic acid solution in DMF (5 equivalents), 0.5M HATU solution in DMF (5 equivalents), and 1M DIEA solution in DMF (10 equivalents) to the solid-phase resin and shaking the resultant mixture at room temperature for 30 minutes.

The deprotection of the side chain and the cleavage from the solid-phase resin were performed as follows. First, the resin obtained after the chloroacetyl group introduction step was washed 5 times with DMF and 3 times with methylene chloride, and then dried under reduced pressure. A reagent cocktail-A (a mixture of TFA/H₂O/TIS/DODT in a volume ratio of 92.5/2.5/2.5/2.5) was then added to the reaction container containing the solid-phase resin and the resultant mixture was shaken at room temperature for 90 minutes. A reaction solution was collected from a frit by filtration. The solid-phase resin remaining in the reaction container was shaken again with a cleavage cocktail, and the solution component was collected from the frit and mixed with the filtrate described above. When the filtrate was added to an excess amount of a cooled diethyl ether/hexane (1/1) solvent mixture, a white precipitate was generated. The mixture was centrifuged, and the solution was decanted. The resultant solid was washed again with a small amount of diethyl ether cooled to 0°C and dried under reduced pressure. The resultant solid was used in the next cyclization reaction.

The cyclization reaction of the peptide was performed by dissolving the peptide in DMSO/H₂O (9/1) such that the final concentration of the peptide reached 2.5 mM based on the number of moles of the solid-phase resin, then adding triethylamine (10 equivalents), and shaking the mixture at room temperature for 4 hours. The resultant reaction solution was concentrated under reduced pressure using a Genevac EZ-2 Elite. The resultant crude product was used in the synthesis of the peptide complex.

The synthesis of the peptide complex was performed by dissolving the peptide in DMSO such that the final concentration of the peptide reached 25 mM based on the number of moles of the solid-phase resin, then adding NHS-cPEG1c-NHS (0.46 equivalents) and DIEA (5 equivalents), shaking the mixture at room temperature for 2 hours, and adding acetic acid.

The resultant crude product was purified under the following conditions. (Column: Waters XBridge (registered trademark) C18, 30 mm × 150 mm; Mobile phase: A= 0.1% TFA in H₂O, B = 0.1% TFA in MeCN; Temperature: 50°C; Gradient (B conc (%)): 5% to 1.1% over 0.1 min, then 1.1% over 4.9 min, then 1.1% to 5% over 1 min, then 5% to 30.6% over 3 min, then 30.6% to 35.7% over 9 min, and then 35.7% to 60% over 1 min; Flow rate: 0.1 min (44 mL/min to 9 mL/min), then 4.9 min (9 mL/min), then 1 min (9 mL/min to 44 mL/min), and then (44 mL/min).)

The purity of the target product calculated from an area ratio in an LC/MS (UV wavelength: 225 nm) chromatogram under the following analysis conditions was 79.87%.

Analysis conditions: Retention time = 3.75 min; Column: Kinetex EVO C18, 2.6 µm, 2.1 mm × 150 mm, 100 Å; Mobile phase: A= 0.025% TFA in H₂O, B = 0.025% TFA in MeCN; Temperature: 60°C; Gradient (B conc (%)): 20% to 60% over 7.15 min, then 60% to 95% over 0.30 min, and then 95% to 95% over 1.55 min; Flow rate: 0.5 mL/min.

ESI-MS(+): Observed m/z value = 1341.8 (M + 4H)⁴⁺.

### Example 5: Synthesis of various peptides

In the present example, various peptide complexes were chemically synthesized as in Examples 1 to 4. Table 1 shows the sequences of synthesized cyclic peptides, Table 2 shows linker structures, and Table 3 shows peptide complexes obtained by the dimerization of the cyclic peptides via linkers.

The synthesized peptide complexes were analyzed under analysis conditions described in Examples 1 to 4, and the structures thereof were checked by ESI-MS(+) in mass spectrometry. Table 3 shows the obtained ESI-MS(+) observed values and retention times, as well as valences.

**[Table 1-1]**

| Peptide Sequence No. | Peptide Sequence | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| 3 | F | L | P | Y | 3Py | W | P | T | Y | S | L | 3Py | V | W | C | G |
| 4 | F | E | P | Y | 3Py | W | P | T | Y | S | L | 3Py | V | W | C | G |
| 5 | F | S | P | Y | 3Py | W | P | T | Y | S | L | 3Py | V | W | C | G |
| 6 | F | L | P | Y | 3Py | W | P | T | Y | S | E | 3Py | V | W | C | G |
| 7 | F | L | P | Y | 3Py | W | P | T | Y | S | L | 3Py | V | P | C | G |
| 8 | F | L | P | Y | 3Py | W | P | T | Y | S | L | 3Py | V | Y | C | G |
| 9 | F | L | P | Y | 3Py | W | P | T | Y | S | L | 3Py | V | S | C | G |
| 10 | F | L | P | Y | 3Py | W | P | T | Y | S | L | 3Py | V | A | C | G |
| 11 | F | L | P | Y | 3Py | W | P | T | Y | S | L | 3Py | V | E | C | G |
| 12 | F | S | P | Y | 3Py | W | P | T | Y | S | E | 3Py | V | W | C | G |
| 13 | F | S | P | Y | 3Py | W | P | T | Y | S | L | 3Py | V | E | C | G |
| 14 | F | L | P | Y | 3Py | W | P | T | Y | S | E | 3Py | V | E | C | G |
| 15 | F | S | P | Y | 3Py | W | P | T | Y | S | E | 3Py | V | E | C | G |

**[Table 1-2]**

| Peptide Sequence No. | Peptide Sequence | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| 16 | F | E | P | Y | 3Py | W | P | T | Y | S | L | 3Py | V | S | C | G |
| 17 | F | E | P | Y | 3Py | W | P | T | Y | S | E | 3Py | V | E | C | G |
| 18 | F | E | P | Y | 3Py | W | P | T | Y | S | E | 3Py | V | S | C | G |
| 19 | F | S | P | Y | 3Py | W | P | S | Y | S | L | 4Py | V | E | C | G |
| 20 | F | S | P | Y | 3Py | W | P4Sh | S | Y | S | L | 4Py | V | E | C | G |
| 21 | F | S | P4Sh | Y | 3Py | W | P4Sh | S | Y | S | L | 4Py | V | E | C | G |
| 22 | F | D | P | Y | 3Py | W | P4Sh | S | Y | S | L | 4Py | V | E | C | G |
| 23 | F | S | P | F4COO | 3Py | W | P4Sh | S | Y | S | L | 4Py | V | E | C | G |
| 24 | F | S | P4Sh | F4COO | 3Py | W | P4Sh | S | Y | S | L | 4Py | V | E | C | G |
| 25 | F | D | P4Sh | F4COO | 3Py | W | P4Sh | S | Y | S | L | 4Py | V | E | C | G |
| 26 | F | Q | P | Y | 3Py | W | P | T | Y | S | L | 3Py | V | E | C | G |
| 27 | F | Cit | P | Y | 3Py | W | P | T | Y | S | L | 3Py | V | E | C | G |
| 28 | F | S | P | Y | 3Py | W | P | T | Y | SMe | L | 3Py | V | E | C | G |
| 29 | F | L | P | Y | 3Py | W | P | T | Y | S | E | 3Py | V | E | C | G |
| 30 | 4Py | L | P | Y | 3Py | W | P | T | Y | S | E | 3Py | V | E | C | G |

**Table 1-3]**

| Peptide Sequence No. | Peptide Sequence | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| 31 | F | Q | P | Y | 3Py | W | P | T | Y | S | E | 3Py | V | E | C | G |
| 32 | F | Cit | P | Y | 3Py | W | P | T | Y | S | E | 3Py | V | E | C | G |
| 33 | F | L | P | F4CON | 3Py | W | P | T | Y | S | E | 3Py | V | E | C | G |
| 34 | F | L | P | Y | 3Py | W | P | T | Y | S | Hgl | 3Py | V | E | C | G |
| 35 | F | L | P | Y | 3Py | W | P | T | Y | S | Atp | 3Py | V | E | C | G |
| 36 | F | L | P | Y | 3Py | W | P | T | Y | S | KAc | 3Py | V | E | C | G |
| 37 | F | L | P | Y | 3Py | W | P | T | Y | S | A4paa | 3Py | V | E | C | G |
| 38 | F | S | P | Y | 3Py | W | P | T | Y | SMe | E | 3Py | V | E | C | G |
| 39 | F | Q | P | Y | 3Py | W | P | T | Y | SMe | E | 3Py | V | E | C | G |
| 40 | F | Q | P | Y | 3Py | W | P | T | Y | S | Hgl | 3Py | V | E | C | G |
| 41 | 4Py | Q | P4Sh | F4CON | 3Py | W | P | S | Y | SMe | A4paa | 4Py | V | E | C | G |

**Table 2**

| Linker Structure No. | Linker Sequence | | | | |
|---|---|---|---|---|---|
| 1 | PEG4c | K | PEG4c | | |
| 2 | PEG8c | K | PEG8c | | |
| 3 | PEG12c | K | PEG12c | | |
| 4 | PEG8c | K | cPEG1c | K | PEG8c |
| 5 | K | cPEG5c | K | | |
| 6 | K | cPEG9c | K | | |
| 7 | K | cPEG13c | K | | |
| 8 | K | cPEG17c | K | | |
| 9 | dk | cPEG17c | dk | | |
| 10 | PEG2c | K | cPEG1c | K | PEG2c |
| 11 | PEG4c | K | cPEG1c | K | PEG4c |
| 12 | PEG6c | K | cPEG1c | K | PEG6c |
| 13 | PEG8c | dk | cPEG1c | dk | PEG8c |
| 14 | K | OCOPEG13OCO | K | | |

**[Table 3-1]**

| Dimer Structure No. | Peptide Sequence No. | Linker Structure No. | Ret time | ESI (m/z) | [M+XH]X+ | Basic Analyzer and Basic Conditions 1 |
|---|---|---|---|---|---|---|
| | | | | | | Slope B (%) |
| 1 | 3 | 1 | 4.67 | 1186.06 | 4 | X |
| 2 | 3 | 2 | 4.82 | 1274.10 | 4 | X |
| 3 | 3 | 3 | 4.95 | 1362.22 | 4 | X |
| 4 | 4 | 2 | 4.34 | 1282.13 | 4 | X |
| 5 | 5 | 2 | 4.36 | 1261.04 | 4 | X |
| 6 | 6 | 2 | 4.38 | 1282.06 | 4 | X |
| 7 | 7 | 2 | 4.27 | 1229.89 | 4 | X |
| 8 | 8 | 2 | 4.28 | 1262.94 | 4 | X |
| 9 | 9 | 2 | 4.19 | 1224.60 | 4 | X |
| 10 | 10 | 2 | 4.38 | 1216.53 | 4 | X |
| 11 | 11 | 2 | 4.20 | 1245.62 | 4 | X |
| 12 | 12 | 2 | 3.85 | 1269.02 | 4 | X |
| 13 | 13 | 2 | 3.60 | 1232.83 | 4 | X |
| 14 | 14 | 2 | 3.80 | 1253.53 | 4 | X |
| 15 | 15 | 2 | 5.80 | 1240.87 | 4 | Y |
| 16 | 16 | 2 | 3.57 | 1232.83 | 4 | X |
| 17 | 17 | 2 | 3.25 | 1261.53 | 4 | X |
| 18 | 18 | 2 | 3.11 | 1240.81 | 4 | X |
| 19 | 19 | 2 | 3.64 | 1225.60 | 4 | X |
| 20 | 20 | 2 | 3.43 | 1233.61 | 4 | X |

**Table 3-2]**

| Dimer Structure No. | Peptide Sequence No. | Linker Structure No. | Ret time | ESI (m/z) | [M+XH]X+ | Basic Analyzer and Basic Conditions 1 |
|---|---|---|---|---|---|---|
| | | | | | | Slope B (%) |
| 21 | 21 | 2 | 3.19 | 1241.60 | 4 | X |
| 22 | 22 | 2 | 3.41 | 1247.53 | 4 | X |
| 23 | 23 | 2 | 3.32 | 1247.52 | 4 | X |
| 24 | 24 | 2 | 3.11 | 1255.41 | 4 | X |
| 25 | 25 | 2 | 3.27 | 1269.64 | 4 | X |
| 26 | 19 | 4 | 3.48 | 1293.34 | 4 | X |
| 27 | 22 | 4 | 5.88 | 1315.76 | 4 | Y |
| 28 | 25 | 4 | 3.12 | 1337.36 | 4 | X |
| 29 | 26 | 2 | 3.76 | 1253.17 | 4 | X |
| 30 | 27 | 2 | 3.78 | 1267.66 | 4 | X |
| 31 | 28 | 2 | 3.80 | 1239.94 | 4 | X |
| 32 | 29 | 4 | 3.72 | 1321.41 | 4 | X |
| 33 | 15 | 4 | 3.18 | 1308.38 | 4 | X |
| 34 | 30 | 4 | 5.37 | 1321.89 | 4 | Y |
| 35 | 31 | 4 | 3.09 | 1328.90 | 4 | X |
| 36 | 32 | 4 | 3.08 | 1343.40 | 4 | X |
| 37 | 33 | 4 | 3.70 | 1335.00 | 4 | X |
| 38 | 34 | 4 | 3.72 | 1328.28 | 4 | X |
| 39 | 35 | 4 | 3.86 | 1334.29 | 4 | X |
| 40 | 36 | 4 | 3.75 | 1341.82 | 4 | X |

**[Table 3-3]**

| Dimer Structure No. | Peptide Sequence No. | Linker Structure No. | Ret time | ESI (m/z) | [M+XH]X+ | Basic Analyzer and Basic Conditions 1 |
|---|---|---|---|---|---|---|
| | | | | | | Slope B (%) |
| 41 | 37 | 4 | 3.44 | 1362.86 | 4 | X |
| 42 | 38 | 4 | 3.55 | 1315.43 | 4 | X |
| 43 | 39 | 4 | 3.46 | 1335.95 | 4 | X |
| 44 | 40 | 4 | 3.14 | 1335.90 | 4 | X |
| 45 | 41 | 4 | 4.20 | 928.95 | 6 | Y |
| 46 | 15 | 5 | 3.45 | 1140.65 | 4 | X |
| 47 | 15 | 6 | 3.58 | 1184.68 | 4 | X |
| 48 | 15 | 7 | 6.02 | 1228.75 | 4 | Y |
| 49 | 15 | 8 | 3.81 | 1272.83 | 4 | X |
| 50 | 15 | 9 | 3.81 | 1272.81 | 4 | X |
| 51 | 15 | 10 | 2.90 | 1176.14 | 4 | X |
| 52 | 15 | 11 | 3.00 | 1220.18 | 4 | X |
| 53 | 15 | 12 | 3.10 | 1264.27 | 4 | X |
| 54 | 15 | 13 | 3.22 | 1308.30 | 4 | X |
| 55 | 15 | 14 | 5.96 | 1214.71 | 4 | Y |
| 56 | 17 | 7 | 5.86 | 1249.73 | 4 | Y |
| 57 | 17 | 14 | 5.95 | 1235.71 | 4 | Y |

### Example 6: Evaluation of TrkB agonist activity by pERK AlphaLISA assay

To evaluate the TrkB activation potency of the TrkB agonist peptide of the present invention, the phosphorylation of ERK was examined. CellSensor (registered trademark) TrkB-NFAT-bla CHO-K1 Cell (Invitrogen) was cultured in DMEM, high glucose, GlutaMAX (registered trademark) Supplement, and pyruvate (Thermo), comprising 10% FBS (Thermo) and 1 × Non-essential amino acids (Thermo), 25 mM HEPES (Thermo), 50 ug/mL Gentamicin, 5 ug/mL Blasticidin (Thermo), and 200 ug/mL Zeocin (Thermo). After the cells were detached using Accutase (Innovative Cell Technologies), they were seeded into a 96-well plate for adherent cultures at 12,500 cells per well and cultured overnight. The next day, for starvation, the culture solution above was replaced with a nutrient starvation medium comprising 0.1% BSA (Sigma), wherein FBS, Blasticidin, and Zeocin were removed from the culture medium, and culturing was performed for 4 hours. Thereafter, Recombinant Human BDNF Protein (R&D Systems) or a peptide was added, and after 15 minutes of stimulation, cells were lysed in the Lysis Buffer provided with the AlphaLISA SureFire Ultra p-Erk1/2 (Thr202/Tyr204) assay kit (PerkinElmer). The assay was performed according to the kit protocol. The signals were detected using a SpectraMax Paradigm Multi-Mode Microplate Reader (Molecular Devices). The obtained signals were analyzed with GraphPad Prism, and the activity (%) was calculated with the maximum value of the signals induced by BDNF as 100% and a signal with no stimulation as 0%. The peptide with 50% or more activity when 1 nM of the peptide was added was evaluated as A, and the peptide with 50% or more activity when 10 nM of the peptide was added was evaluated as B.

The BDNF was evaluated at 8 points (0.0006 nM to 10 nM) from 10 nM using 4-fold dilutions.

For dimer structures Nos. 1 to 3, the peptide was evaluated at 1 nM, 10 nM, and 100 nM, and, for other dimer structure numbers, the peptide was evaluated at 0.1 nM, 1 nM, and 10 nM. When creating the concentration dependence curve, the BDNF and the peptides were evaluated at 8 points (0.006 nM to 100 nM) from 100 nM using 4-fold dilutions.

The results are shown in Table 4 and FIG. 1. As shown in Table 4, the synthesized peptide complexes were demonstrated to have TrkB agonist activity. As shown in FIG. 1, the synthesized peptide complexes (dimer structures Nos. 48 and 56 in Table 3) were demonstrated to have similar TrkB agonist activity to the brain-derived neurotrophic factor (BDNF).

**Table 4-1]**

| Dimer Structure Number | pERK (aLISA) Assay Results | NFAT Assay Results |
|---|---|---|
| 1 | B | C |
| 2 | B | B |
| 3 | B | B |
| 4 | B | B |
| 5 | B | not tested |
| 6 | A | A |
| 7 | B | A |
| 8 | B | not tested |
| 9 | B | A |
| 10 | B | B |
| 11 | A | A |
| 12 | A | A |
| 13 | A | A |
| 14 | A | A |
| 15 | A | A |
| 16 | A | A |
| 17 | A | A |
| 18 | A | A |
| 19 | A | A |
| 20 | A | A |

**Table 4-2]**

| Dimer Structure Number | pERK (aLISA) Assay Results | NFAT Assay Results |
|---|---|---|
| 21 | A | A |
| 22 | A | A |
| 23 | A | A |
| 24 | A | A |
| 25 | A | A |
| 26 | A | A |
| 27 | A | A |
| 28 | A | A |
| 29 | A | A |
| 30 | A | A |
| 31 | A | A |
| 32 | A | A |
| 33 | A | A |
| 34 | A | A |
| 35 | A | A |
| 36 | A | A |
| 37 | A | A |
| 38 | A | A |
| 39 | A | A |
| 40 | A | A |

**Table 4-3]**

| Dimer Structure Number | pERK (aLISA) Assay Results | NFAT Assay Results |
|---|---|---|
| 41 | A | A |
| 42 | A | A |
| 43 | A | A |
| 44 | A | A |
| 45 | A | A |
| 46 | A | A |
| 47 | A | A |
| 48 | A | A |
| 49 | A | A |
| 50 | A | A |
| 51 | A | A |
| 52 | A | A |
| 53 | A | A |
| 54 | A | A |
| 55 | A | A |
| 56 | A | A |
| 57 | A | A |

### Example 7: Evaluation of TrkB agonist activity by NFAT assay

To evaluate the TrkB activation potency of the TrkB agonist peptide of the present invention, transcriptional activity was examined using transcriptional reporters. The CellSensor (registered trademark) TrkB-NFAT-bla CHO-K1 Cell (Invitrogen) used in the AlphaLISA assay was cultured in the medium above. After the cells were detached using Accutase (Innovative Cell Technologies), the cells were suspended in an assay medium, wherein Blasticidin and Zeocin were removed, then seeded into a 96-well plate for adherent cultures at 12,500 cells per well and cultured overnight. Then, BDNF or a peptide was added thereto, and the cells were further cultured in a CO₂ incubator for 5 hours. After the 96-well plate was removed from the incubator and brought to room temperature, a detection reagent (LiveBLAzer (registered trademark)-FRET B/G (CCF4-AM) Substrate Mixture) was added while protected from the light, and the 96-well plate was incubated while protected from the light in a moisture box with a lid for 2 hours. For signal detection, a EnVision 2104 Multilabel Plate Reader (PerkinElmer) was used, and the ratio of fluorescence emission value (460 nm/535 nm) was calculated. The obtained numerical values were analyzed with GraphPad Prism, and the activity (%) was calculated with the maximum value of the signals induced by BDNF as 100% and a signal with no stimulation as 0%. The peptide with 50% or more activity when 1 nM of the peptide was added was evaluated as A, the peptide with 50% or more activity when 10 nM of the peptide was added was evaluated as B, and the peptide with 50% or more activity when 100 nM of the peptide was added was evaluated as C. Those that were not evaluated were listed as not tested.

The BDNF was evaluated at 7 points (0.0024 nM to 10 nM) from 10 nM using 4-fold dilutions.

For dimer structures Nos. 1 to 3, the peptide was evaluated at 1 nM, 10 nM, and 100 nM, and, for other dimer structure numbers, the peptide was evaluated at 0.1 nM, 1 nM, and 10 nM. When creating the concentration dependence curve, the BDNF and the peptides were evaluated at 7 points (0.006 nM to 100 nM) from 25 nM using 4-fold dilutions.

The results are shown in Table 4 and FIG. 2. As shown in Table 4, the synthesized peptide complexes were demonstrated to have TrkB agonist activity. As shown in FIG. 2, the synthesized peptide complexes (dimer structures Nos. 48 and 56 in Table 3) were demonstrated to have similar TrkB agonist activity to the brain-derived neurotrophic factor (BDNF).

### Example 8: Evaluation of TrkB agonist activity by PathHunter assay

To evaluate the TrkB activation potency of the TrkB agonist peptide of the present invention, the phosphorylation of TrkB was examined. The PathHunter (registered trademark) eXpress TrkB Functional Assay kit (DiscoverX; cells, 96-well plate, medium, and detection reagents included) was used and the evaluation was performed according to the attached protocols. The frozen cells were thawed and suspended in the medium, then seeded into a 96-well plate at 10,000 cells per well and incubated for 48 hours. Thereafter, Recombinant Human BDNF Protein (R&D Systems) or a peptide was added, and after stimulation for 3 hours, a detection reagent was added. The reaction was performed while protected from the light at room temperature for 60 minutes, and luminescent signals were measured using a SpectraMax Paradigm Multi-Mode Microplate Reader (Molecular Devices). The obtained signals were analyzed with GraphPad Prism, and the activity (%) was calculated with the maximum value of the signals induced by BDNF as 100% and a signal with no stimulation as 0%.

The BDNF was evaluated at 8 points (0.006 nM to 100 nM) from 100 nM using 4-fold dilutions. The peptide was evaluated in the measurement ranges A to C shown below.
Measurement range A = 6 points from 100 nM using 5-fold dilutions (0.032 nM to 100 nM)
Measurement range B = 8 points from 100 nM using 4-fold dilutions (0.006104 nM to 100 nM)
Measurement range C = 4 points from 10 nM using 10-fold dilutions (0.01 nM to 10 nM)

The results are shown in Table 5 and FIG. 3. As shown in Table 5, the synthesized peptide complexes were demonstrated to have TrkB agonist activity. As shown in FIG. 3, the synthesized peptide complexes (dimer structures Nos. 48 and 56 in Table 3) were demonstrated to have similar TrkB agonist activity to the brain-derived neurotrophic factor (BDNF).

**Table 5]**

| Dimer Structure Number | PathHunter | | |
|---|---|---|---|
| | EC50 (nM) | Emax | Measurement range |
| 2 | 0.8-4 | 89% | A |
| 3 | 0.8-4 | 89% | A |
| 14 | 0.197 | 86% | A |
| 15 | 0.290 | 104% | A |
| 18 | 0.260 | 93% | A |
| 33 | 0.354 | 96% | A |
| 47 | 0.556 | 107% | A |
| 49 | 0.644 | 107% | A |
| 51 | 0.599 | 106% | A |
| 48 | 0.421 | 102% | B |
| 56 | 0.358 | 93% | B |
| 55 | 0.581 | 104% | C |
| 57 | 0.669 | 99% | C |

### Example 9: Evaluation of intermolecular interactions between the peptide and TrkA, TrkB, and TrkC using surface plasmon resonance (SPR)

In the present example, for synthesized peptide complexes (dimer structures Nos. 48 and 56 in Table 3), molecular interactions between the peptide and TrkA, TrkB, and TrkC was evaluated using surface plasmon resonance (SPR) by the following method.

### [SPR measurement]

A CM3 sensor chip (Cytiva) was inserted into a Biacore T200 (Cytiva). Three priming runs were performed using running buffer: HBS-EP+ (Cytiva). The system was then equilibrated at a flow rate of 30 µL/min. After mixing in 50 µL each of a 60 mM EDC solution (Cytiva) and 650 mM NHS solution (Cytiva), the reaction was performed at a flow rate of 10 µL/min for 420 seconds. Then, 135 µL of a 30 ng/uL anti-human IgG (Fc) antibody (Cytiva) solution was prepared by dilution with a 10 mM acetic acid solution (pH 5.0), and the anti-human IgG (Fc) antibody was immobilized on the CM3 sensor chip by reacting it at a flow rate of 10 µL/min for 360 seconds. After immobilization, capping was performed by reacting it with a 1.0M ethanolamine solution (Cytiva) at a flow rate of 10 µL/min for 420 seconds. Each 200nM Recombinant Human TrkA Fc Chimera Protein, Recombinant Human TrkB Fc Chimera Protein, Recombinant Human TrkC Fc Chimera Protein (R&D Systems) solution was reacted with the immobilized antibody at a flow rate of 10 µL/min for 60 seconds. A liquid prepared by dissolving each peptide at 10 mM in DMSO solution was diluted with running buffer to prepare a peptide-dissolved solution at a final concentration of 10 µM. Subsequently, 50 nM, 25 nM, 12.5 nM, 5 nM, and 2.5 nM peptide solutions were prepared. Using the samples described above, the kinetics of peptide against TrkA, TrkB, and TrkC were obtained by SPR measurements. After reacting a peptide with TrkA, TrkB, and TrkC, regeneration was performed using a reaction with a 3M MgCl solution at 10 µL/min for 180 seconds.

The kinetic evaluation model was Single Cycle Kinetics, and curve fitting was performed using Biacore T200 Evaluation Software Version 3.0 (Cytiva). The binding of peptides to TrkA, TrkB, and TrkC was evaluated by performing least-squares curve fitting on the obtained sensorgrams and calculating their Kd values (dissociation constants).

The results are shown in FIG. 4. As shown in FIG. 4, the synthesized peptide complexes (dimer structures Nos. 48 and 56 in Table 3) showed no binding to TrkA and TrkC, and only showed binding to TrkB. For the binding of the synthesized peptide complexes to TrkB, the koff (dissociation rate constant) exceeded the measurement limit. Thus, Kd was calculated by employing the lower theoretical limit of 0.00001 (1/s) for koff. Kd was 100 pM or less.

### INDUSTRIAL APPLICABILITY

This invention may be used in the pharmaceutical and bioengineering industries.

## Claims

1. A peptide complex comprising a first peptide and having TrkB binding activity, wherein
the first peptide:
has an amino acid sequence represented by X¹-X²-X³-X⁴-3Py-W-X⁵-X⁶-X⁷-X⁸-X⁹-X¹⁰-V-X¹¹-C (SEQ ID NO: 1); or
consists of an amino acid sequence with substitution, deletion, addition, or insertion of 1 to 4 amino acids in the amino acid sequence represented by SEQ ID NO: 1,
X¹ is an amino acid having an aromatic ring in a side chain,
X² is any amino acid,
X³ is a secondary amino acid,
X⁴ is an amino acid having an aromatic ring in a side chain, wherein the aromatic ring may be substituted,
X⁵ is a secondary amino acid,
X⁶ is an amino acid having a chain alkyl group in a side chain,
X⁷ is an amino acid having an aromatic ring in a side chain, wherein the aromatic ring may be substituted,
X⁸ is an amino acid having a chain alkyl group in a side chain,
X⁹ is any amino acid,
X¹⁰ is an amino acid having an aromatic ring in a side chain, and
X¹¹ is any amino acid.

2. The peptide complex according to claim 1, wherein
X² is an amino acid having a chain or cyclic alkyl group in a side chain,
X³ is proline or a proline derivative,
X⁴ is tyrosine or an amino acid in which a hydroxyl group of tyrosine is substituted with another functional group,
X⁵ is proline or a proline derivative,
X⁶ is an amino acid having a chain alkyl group and a polar group in a side chain,
X⁷ is tyrosine or an amino acid in which a hydroxyl group of tyrosine is substituted with another functional group,
X⁸ is an amino acid having a chain alkyl group and a polar group in a side chain, and
X⁹ is an amino acid having a functional group with 3 or more carbon atoms in a side chain.

3. The peptide complex according to claim 2, wherein
X² is an amino acid further having a polar group in a side chain,
X⁶ is an amino acid having a chain alkyl group and a hydroxyl group in a side chain,
X⁷ is tyrosine or an amino acid in which a hydroxyl group of tyrosine is substituted with another functional group,
X⁸ is an amino acid having a chain alkyl group and a hydroxyl group which may be substituted in a side chain, and
X⁹ is an amino acid having a chain or cyclic alkyl group in a side chain.

4. The peptide complex according to claim 1, wherein
X¹ is 4Py or F,
X² is alT, Cit, D, E, L, Q, or S,
X³ is P or P4Sh,
X4 is F4CON, F4COO, or Y,
X⁵ is HyP, P, or P4Sh,
X⁶ is S or T,
X⁷ is F4CON or Y,
X⁸ is S or SMe,
X⁹ is A4paa, Atp, E, Hgl, KAc, or L,
X¹⁰ is 3Py or 4Py, and
X¹¹ is A, E, MeE, P, S, or W.

5. The peptide complex according to claim 1, wherein
the first peptide:
has an amino acid sequence represented by F-L-P-Y-3Py-W-P-T-Y-S-L-3Py-V-W-C (SEQ ID NO: 2); or
consists of an amino acid sequence with substitution, deletion, addition, or insertion of 1 to 3 amino acids in the amino acid sequence represented by SEQ ID NO: 2.

6. The peptide complex according to claim 1, wherein
the first peptide has an amino acid sequence with an addition of glycine at the C-terminus.

7. The peptide complex according to claim 1, wherein
the first peptide is a peptide consisting of an amino acid sequence represented by any one of SEQ ID NOs: 2 to 41.

8. The peptide complex according to claim 1, wherein
the first peptide is a cyclic peptide.

9. The peptide complex according to claim 1, wherein
the peptide complex has TrkB agonist activity.

10. The peptide complex according to claim 1, wherein
the first peptide is a cyclic peptide having a chloroacetylated amino-acid residue as an amino-acid residue at the N-terminus and a cysteine residue in the peptide, wherein the amino-acid residue at the N-terminus and the cysteine residue are bonded to each other.

11. The peptide complex according to claim 1, wherein
the peptide complex comprises: the first peptide; a second peptide; and a linker connecting the first peptide and the second peptide,
the second peptide is identical to or different from the first peptide and has an amino acid sequence represented by SEQ ID NO: 1 or consists of an amino acid sequence with substitution, deletion, addition, or insertion of 1 to 4 amino acids in the amino acid sequence represented by SEQ ID NO: 1.

12. The peptide complex according to claim 11, wherein
a homology between the first peptide and the second peptide is from 80% to 100% inclusive.

13. The peptide complex according to claim 11, wherein
the first peptide and the second peptide are homodimers which are substantially the same.

14. The peptide complex according to claim 11, wherein
the linker is a PEG linker.

15. A composition comprising:
the peptide complex according to claim 1; and
a carrier.

16. A composition comprising:
the peptide complex according to claim 1; and
a carrier, wherein
the composition is used for medical, diagnostic, or research purposes.

17. A cell culture composition comprising:
the peptide complex according to claim 1; and
a carrier, wherein
the cell culture composition is used for cell culture.
